# EUROPÄISCHE PATENTSCHRIFT

(11) **EP 3 802 917 B1**
(45) Veröffentlichungstag und Bekanntmachung des Hinweises auf die Patenterteilung: **06.07.2022**
(21) Anmeldenummer: 19729648.6
(22) Anmeldetag: 28.05.2019
(51) Int. Cl.: C25B 1/04, C25B 15/08, C07D 307/46

(54) **ANOLYTFRAKTION-KATALYSIERTE HMF-HERSTELLUNG**
HMF PREPARATION CATALYSED BY ANOLYTE FRACTION
PRODUCTION DE HMF CATALYSÉE PAR FRACTION D'ANOLYTE

(30) Priorität: 29.05.2018 DE 102018208507
(43) Veröffentlichungstag der Anmeldung: 14.04.2021
(60) Teilanmeldung: 22175244.7
(73) Patentinhaber: Südzucker AG, 68165 Mannheim (DE)
(72) Erfinder: HAJI BEGLI, Alireza, 67305 Ramsen (DE); KRÖNER, Christine, 67271 Kindenheim (DE); TSCHILINGIRI, Waldemar, 67549 Worms (DE); RIEMENSCHNITTER, Ralf, 67316 Carlsberg (DE); MANTYK, Kay, 67283 Obrigheim (DE)
(74) Vertreter: Schrell, Andreas
(86) Internationale Anmeldenummer: PCT/EP2019/063851
(87) Internationale Veröffentlichungsnummer: WO 2019/229077

(56) Entgegenhaltungen:
- US-A- 4 395 316
- US-A1- 2011 272 291
- US-A1- 2014 315 262

## Beschreibung

Die vorliegende Erfindung betrifft ein Verfahren zur Herstellung von 5-Hydroxymethylfurfural (HMF), das eine Fructose-haltige Komponente unter Verwendung einer katalytisch aktiven Anolytfraktion, die durch Elektrolyse von Wasser hergestellt worden ist, bei einer Temperatur von 90 bis 200 °C umsetzt und zum Erhalten einer HMF-haltigen Produktmischung führt, wobei vorteilhafterweise eine hohe HMF-Selektivität bei gleichzeitig deutlich geringerer Nebenproduktbildung erreicht wird.

5-Hydroxymethylfurfural (HMF) ist ein multifunktionales Molekül mit einem aromatischen 5-Ring-System, einer Aldehyd- und einer Alkoholgruppe. Die vielen Funktionalitäten machen das Molekül zu einer vielfältig einsetzbaren Plattformchemikalie, die als Basis für eine große Vielzahl weiterer Verbindungen dienen kann. Zu den Verbindungen, die auf Basis von HMF hergestellt werden können, gehören zum einen Chemikalien, die heute bereits im Bulkmaßstab auf petrochemischem Wege hergestellt werden, beispielsweise Caprolactam oder Adipinsäure, aber auch Verbindungen mit einem großen Anwendungspotential, für die bislang noch kein technischer Herstellprozess zur Verfügung steht, wie 2,5-Furandicarbonsäure (FDCA).

Trotz des großen Potentials von HMF und FDCA fehlt es bislang an wirtschaftlichen, technisch etablierten Herstellungsverfahren für diese Verbindungen. Die Multifunktionalität von HMF als einer der größten Vorteile des Moleküls hat sich in Bezug auf die daraus resultierende mögliche Folgechemie gleichfalls als Hauptnachteil bei der Synthese erwiesen. HMF ist vor allem in wässrigen Systemen unter den für die Synthese notwendigen Reaktionsbedingungen (saurer pH-Wert, erhöhte Temperatur) nicht stabil und reagiert zum einen unter Polymerisation mit sich selbst und/oder den Edukten und Zwischenprodukten zu sogenannten Huminen, die je nach Kettenlänge löslich oder unlöslich sind und zu einer Braun- bis Schwarzfärbung der Reaktionslösung führen. Eine weitere unerwünschte Folgereaktion ist die sauerkatalysierte Rehydratisierung von HMF zu Lävulin- und Ameisensäure, wobei insbesondere Lävulinsäure mit HMF zu weiteren unerwünschten Nebenprodukten reagieren kann. Für eine möglichst wirtschaftliche Herstellung von HMF ist es daher unbedingt notwendig, das Auftreten dieser Nebenreaktion sowie die Folgereaktion von HMF und Lävulinsäure soweit wie möglich zu vermeiden.

Grundsätzlich kann bei den vielzähligen unterschiedlichen Synthesewegen, die im Stand der Technik zur Herstellung von HMF beschrieben wurden, zwischen einphasigen und zweiphasigen Reaktionssystemen unterschieden werden. Bei beiden Ansätzen können sowohl homogene als auch heterogene Katalysatoren zum Einsatz kommen. Bei den einphasigen Systemen kann die HMF-Synthese neben rein wässrigen Systemen auch in organischen Lösungsmitteln, wie beispielsweise DMSO, DMF und Sulfolan, oder in ionischen Flüssigkeiten durchgeführt werden. Die Vermeidung von wässrigen Systemen führt zwar rein auf die chemische Reaktion bezogen zu besseren Selektivitäten für HMF, jedoch sind für die Entfernung der Lösungsmittel oftmals hohe Temperaturen notwendig, unter denen es zur thermischen Zersetzung von HMF kommen kann, wodurch wiederum die Reinheit und Ausbeute des HMF deutlich verschlechtert wird. Zudem spielen bei der Verwendung von wasserfreien Systemen die Kosten für die Lösungsmittel sowie Sicherheits- und Umweltaspekte eine große Rolle. Auch erweist sich als nachteilig, dass die für die HMF-Synthese eingesetzten Hexosen, insbesondere Fructose und/oder Glucose, in vielen gängigen organischen Lösungsmitteln eine schlechte Löslichkeit aufweisen.

Bei den zweiphasigen Reaktionssystemen wird die Reaktion der Hexose zu HMF in wässriger Phase durchgeführt und das entstandene HMF kontinuierlich mithilfe eines organischen Lösungsmittels extrahiert. Dabei darf das Lösungsmittel nicht mit Wasser mischbar sein und muss einen ausreichend hohen Verteilungskoeffizienten für HMF zwischen wässriger und organischer Phase aufweisen, um eine effiziente Extraktion des HMF zu gewährleisten. Da insbesondere die Verteilungskoeffizienten für die meisten Lösungsmittel nicht sehr hoch sind, müssen in solchen Systemen oftmals sehr große Mengen an Lösungsmittel eingesetzt werden. Das in zweiphasigen Reaktionssystemen am häufigsten verwendete organische Lösungsmittel ist dabei Methylisobutylketon (MIBK), welches gegebenenfalls in Kombination mit Phasenmodifizierern wie 2-Butanol eingesetzt wird. Wie bereits für die einphasigen wasserfreien Reaktionssysteme dargestellt, erweist sich jedoch auch in diesem Fall die abschließende Entfernung der oder des verwendeten Lösungsmittel(s) aufgrund der hohen Siedepunkte geeigneter Lösungsmittel als problematisch.

EP 0 230 250 B1 offenbart Verfahren zur Herstellung von 5-Hydroxymethylfurfural einschließlich eines kristallinen Produktes unter alleiniger Verwendung von Wasser als Lösungsmittel. In dem beschriebenen Batch-Verfahren werden Saccharide in wässriger Lösung bei einer Temperatur von über 100 °C mit einem sauren Katalysator zu einem Gemisch von Hexosen und HMF zersetzt und anschließend das gebildete HMF über Ionenaustauschersäulen bei einer Temperatur von 35 bis 85 °C von Nebenprodukten so getrennt, dass neben einer HMF-Fraktion eine Saccharid-Fraktion gewonnen werden kann, die für eine erneute HMF-Synthese gemäß dem beschriebenen Verfahren zur Verfügung steht. Die in diesem Dokument offenbarte batchweise Umsetzung geht mit einem hohen Fructoseumsatz und einer daraus unmittelbar folgenden hohen HMF-Konzentration in der Reaktionslösung einher, die unter den vorherrschenden Bedingungen zu einer vermehrten Bildung von Neben- und Abbauprodukten führt, wodurch es in Bezug auf die umgesetzte Fructosemenge zu einer verringerten HMF-Ausbeute kommt.

WO 2013/106136 A1 betrifft ein Verfahren zur Herstellung von HMF und HMF-Derivaten aus Zucker, umfassend die Rückgewinnung von nicht umgesetzten Zuckern, die zur direkten Verwendung in der Ethanol-Fermentation geeignet sind. Dabei werden Hexose-haltige Lösungen in wässriger Phase durch säurekatalysierte Dehydratisierungsreaktion zu HMF umgesetzt, anschließend werden die in der Produktmischung enthaltenen nicht umgesetzten Zucker durch Adsorption und/oder Lösungsmittelextraktion aus der Produktmischung abgetrennt und diese schließlich in aeroben oder anaeroben Fermentationsverfahren zur Ethanolgewinnung eingesetzt. Es wird gelehrt, die säurekatalysierte Dehydratisierungsreaktion bei einer Temperatur von 175 bis 205 °C durchzuführen.

WO 2015/113060 A2 offenbart die Umwandlung von Fructose-haltigen Ausgangsmaterialien zu HMF-haltigen Produkten. Mittels des beschriebenen Verfahrens werden Fructose, Wasser, ein Säurekatalysator und mindestens ein weiteres Lösungsmittel in einer Reaktionszone vermischt und durch Wahl geeigneter Reaktionsparameter für eine Dauer von ca. 1 bis 60 Minuten zur Reaktion gebracht, sodass eine HMF-Ausbeute von 80 % nicht überschritten wird. Beim Erreichen des festgelegten Umsatzes werden die Reaktionskomponenten umgehend abgekühlt, um die Bildung von unerwünschten Nebenprodukten zu minimieren.

WO 2014/158554 offenbart Verfahren zur Herstellung von HMF oder Derivaten davon aus Glucose- und/oder Fructose-haltigen Lösungen, wobei die säurekatalysierte Dehydratisierungsreaktion unter Sauerstoff-reduzierten Bedingungen durchgeführt wird. Dies soll die Stabilität von HMF erhöhen und mögliche Degradationsreaktionen verhindern, sodass die Bildung von unerwünschten Nebenprodukten reduziert wird. Gegebenenfalls werden Antioxidantien zugegeben, um eine Autooxidationsreaktion von HMF zu verhindern.

Zur Sicherstellung eines kostengünstigen und effektiven Herstellungsverfahrens für HMF ist entscheidend, dass während der Umsetzung einer Fructose-haltigen Ausgangslösung zu HMF die Bildung von unerwünschten Nebenprodukten und die Zersetzung des durch die Dehydratisierungsreaktion gebildeten HMF durch die Wahl geeigneter Reaktionsbedingungen und Verfahrensschritte soweit wie möglich vermieden wird. Weiterhin ist es wirtschaftlich sinnvoll, wenn die nicht umgesetzte Fructose von den während der Dehydratisierungsreaktion gebildeten störenden Nebenprodukten abgetrennt wird und somit in möglichst reiner Form gegebenenfalls für die Rückführung in den kontinuierlichen Herstellungsprozess bereitgestellt wird.

Ein entsprechendes Verfahren zur kostengünstigen und effektiven Herstellung von HMF, bevorzugt in einem kontinuierlichen Prozess, ist bislang aus dem Stand der Technik nicht bekannt.

Es ist daher die Aufgabe der vorliegenden Erfindung, die genannten Nachteile und Einschränkungen der aus dem Stand der Technik bekannten Verfahren zu überwinden, insbesondere ein Verfahren bereitzustellen, Fructose hochselektiv, insbesondere unter maximaler Vermeidung von Nebenproduktbildung und auf kostengünstige und effektive Weise, zu HMF umzusetzen.

Die Aufgabe der vorliegenden Erfindung wird durch die technische Lehre der Ansprüche gelöst. Insbesondere betrifft die vorliegende Erfindung ein Verfahren zur Herstellung von 5-Hydroxymethylfurfural (HMF) umfassend die Schritte:
a) Bereitstellen einer Fructose-haltigen Komponente und einer katalytisch aktiven Anolytfraktion, welche durch Elektrolyse von Wasser hergestellt worden ist, wobei die Elektrolyse von Wasser in einer Elektrolysezelle durchgeführt wurde, worin die Kathode von der Anode getrennt ist, wobei die Anolytfraktion aus dem Anodenraum der Elektrolysezelle nach Durchführung der Elektrolyse entnommen wurde und wobei die katalytisch aktive Anolytfraktion einen Sauerstoffgehalt oberhalb der Sättigungskonzentration aufweist,
b) Vermischen der Fructose-haltigen Komponente und der katalytisch aktiven Anolytfraktion zum Erhalten einer Reaktionslösung,
c) Umsetzen der in der Reaktionslösung vorhandenen Fructose zu HMF bei einer Temperatur von 90 °C bis 200 °C zum Erhalten einer flüssigen HMF-haltigen Produktmischung und
d) Erhalten einer flüssigen HMF-haltigen Produktmischung.

Erfindungsgemäß wird demnach ein Verfahren bereitgestellt, welches 5-Hydroxymethylfurfural (HMF) durch selektive, bevorzugt hochselektive, Umsetzung der Fructose einer Fructose-haltigen Komponente herstellt. Für die Umsetzung der Fructose wird erfindungsgemäß eine katalytisch aktive Anolytfraktion, die durch Elektrolyse von Wasser hergestellt worden ist, eingesetzt. Die vorliegende Erfindung sieht vorteilhafterweise also vor, eine Fructose-haltige Komponente mit einer katalytisch aktiven Anolytfraktion zu vermischen und anschließend eine Umsetzung der in der Reaktionslösung vorhandenen Fructose zu HMF durchzuführen. Der Einsatz einer katalytisch aktiven Anolytfraktion zur Umsetzung der in der Fructose-haltigen Komponente vorhandenen Fructose zu HMF ist insofern vorteilhaft, als dass eine im Vergleich zu üblichen HMF-Herstellungsverfahren, welche beispielsweise Schwefelsäure als Katalysator einsetzen, eine deutlich höhere HMF-Selektivität bei gleichzeitig deutlich verringerter Nebenproduktbildung und vergleichbaren Fructoseumsätzen bereitgestellt wird. So sind in vorteilhafter bevorzugter Ausführungsform Fructoseumsätze ≥ 30% bei akzeptabler Selektivität von mehr als 80% möglich. Darüber hinaus ermöglicht der Einsatz der Anolytfraktion auch eine höhere Kohlenhydratkonzentration in der Reaktionslösung, nämlich in vorteilhafter bevorzugter Ausführungsform bis zu 40% Trockensubstanz Kohlenhydrat. Erfindungsgemäß führt die Verwendung der katalytisch aktiven Anolytfraktion zu sehr hohen HMF-Selektivitäten, ohne dass in vorteilhafter bevorzugter Ausführungsform der Einsatz anderer Katalysatoren in homogener oder heterogener Form notwendig ist. Überraschenderweise konnte gezeigt werden, dass bei der Verwendung der katalytisch aktiven Anolytfraktion als Katalysator für die Umsetzung von Fructose zu HMF in vorteilhafter bevorzugter Ausführungsform ein inverser Zusammenhang zwischen der Fructosereinheit und der HMF-Selektivität besteht, das heißt mit abnehmendem Fructoseanteil an der Kohlenhydratzusammensetzung die Selektivität für HMF steigt. Darüber hinaus führt die Verwendung der katalytisch aktiven Anolytfraktion zu einer deutlich geringeren Huminstoffbildung, insbesondere von unlöslichem Humin, die bei der üblichen Prozessführung durch Anbackungen und Verkrustungen zu technischen Problemen führen. Die erfindungsgemäße Verwendung der katalytisch aktiven Anolytfraktion, welche sauerstoffhaltig ist, führt demgemäß insbesondere zu deutlich höheren Fructoseumsätzen bei wirtschaftlich sinnvoller HMF-Selektivität. Dieser Effekt ist besonders überraschend im Lichte der vorstehend erwähnten WO 2014/158554, da im dort beschriebenen Verfahren insbesondere sauerstoffreduzierte Bedingungen und/oder die Anwesenheit von Antioxidantien zu einer erhöhten HMF-Stabilität führen und mögliche Abbaureaktionen verhindern sollen.

In besonders bevorzugter Ausführungsform ermöglicht es die erfindungsgemäße Verfahrensweise, insbesondere die Durchführung der Verfahrensschritte a) bis d), deutlich höhere HMF-Selektivität zu erreichen, wobei bei vergleichbaren Fructose-Umsätzen im Vergleich zum Stand der Technik eine verringerte Nebenproduktbildung, insbesondere eine verringerte Rehydratisierung von HMF zu Lävulinsäure und Ameisensäure auftritt.

In besonders bevorzugter Ausführungsform beträgt die Selektivität für Lävulinsäure im erfindungsgemäßen Verfahren, insbesondere den Verfahrensschritten a) bis d), ≤ 5 %, bevorzugt ≤ 4 %, bevorzugt ≤ 3 %, besonders bevorzugt ≤ 2 % (bezogen auf den umgesetzten Fructoseanteil).

Im Zusammenhang mit der vorliegenden Erfindung wird unter einer katalytisch aktiven Anolytfraktion die im Zuge und als Ergebnis einer Elektrolyse von Wasser an der Anode erhaltene Wasserfraktion verstanden. Die nach der Elektrolyse des Wassers im Anoden-Raum befindliche Lösung wird erfindungsgemäß als katalytisch aktive Anolytfraktion im vorliegenden Verfahren verwendet. Diese "katalytisch aktive Anolytfraktion" wird vorliegend auch als Anolyt, saures aktiviertes Wasser, elektrolysiertes oxidierendes Wasser, saures elektroaktiviertes Wasser oder saures elektrochemisch-aktiviertes Wasser bezeichnet.

Die Herstellung der katalytisch aktiven Anolytfraktion findet erfindungsgemäß in einer Elektrolysezelle statt, worin die Kathode von der Anode getrennt ist, vorzugsweise durch eine Membran oder ein Diaphragma. Nach Durchführung der Elektrolyse wird die katalytisch aktive Anolytfraktion aus dem Anodenraum der Elektrolysezelle entnommen und im erfindungsgemäßen Verfahren eingesetzt. Das zur Herstellung der katalytisch aktiven Anolytfraktion eingesetzte Wasser ist bevorzugt vollentsalztes Wasser (VE-Wasser), destilliertes Wasser, Trinkwasser oder Leitungswasser. Es kann jedoch auch vorgesehen sein, dass das Wasser für die Elektrolyse ein Salz aufweist. Dieses ist bevorzugt ausgewählt aus der Gruppe bestehend aus LiCl, NaCl, KCl, NaF oder andere Alkali- oder Erdalkalihalogenide, insbesondere Alkali- oder Erdalkalichloride oder Alkali- oder Erdalkalifluoride, NaNO₃ oder andere Alkalioder Erdalkalinitrate, Na₂SO₄ oder andere Alkali- oder Erdalkalisulfate, Natriumcitrat oder andere Salze der Zitronensäure, oder Gemischen davon. Besonders bevorzugt weist das Wasser für die Elektrolyse ein Salz ausgewählt aus der Gruppe bestehend aus Alkalihalogeniden, Erdalkalihalogeniden, Alkalinitraten, Erdalkalinitraten, Alkalisulfaten, Erdalkalisulfaten, Citraten, Acetaten, Tartraten, Oxalaten, Glycolaten, Gluconaten und Gemischen davon auf. Besonders bevorzugt enthält das Wasser für die Elektrolyse ein Salz ausgewählt aus der Gruppe bestehend aus NaCl, NaNO₃, Na₂SO₄, LiCl und KCl. Insbesondere enthält das Wasser bevorzugt NaCl. Das im Wasser für die Elektrolyse enthaltene Salz wird vorliegend auch als Elektrolyt bezeichnet.

Erfindungsgemäß weist das Wasser für die Elektrolyse 0,01 bis 2,5 Gew.-%, bevorzugt 0,05 bis 2,2 Gew.-%, bevorzugt 0,1 bis 2,0 Gew.-%, bevorzugt 0,5 bis 1,5 Gew.-%, besonders bevorzugt 0,1 bis 1 Gew.-% Salz, insbesondere 0,05 Gew.-%, 0,1 Gew.-%, 0,18 Gew.-%, 0,25 Gew.-%, 0,625 Gew.-% oder 1,0 Gew.-% Salz (bezogen auf das Gesamtgewicht des Wassers) auf.

Durch die Elektrolyse von reinem Wasser, z. B. vollentsalztem Wasser oder Leitungswasser, also ohne Zusatz eines Elektrolyten, das heißt eines Salzes, entstehen im Anoden- und Kathodenraum, die vorzugsweise durch eine Membran oder ein Diaphragma voneinander getrennt sind, zwei unterschiedliche Lösungen. An der Anode entstehen aus Hydroxidionen und H₂O Oxoniumionen (H₃O⁺), Elektronen und Sauerstoff. Durch Nebenreaktionen an den Elektroden können in geringen Mengen auch weitere reaktive Spezies wie Ozon (O₃) sowie verschiedene Radikale entstehen wie beispielsweise HO₂^{∗}, OH^{∗} oder auch H₂O^{∗}. Die durch die Elektrolyse von VE-Wasser erhaltene katalytisch aktive Anolytfraktion zeichnet sich daher insbesondere durch die Anwesenheit von O₂ und Oxoniumionen (H₃O⁺) aus. An der Kathode entstehen aus Wasser Hydroxidionen (OH⁻) und Wasserstoff. Auch hier können durch Nebenreaktionen weitere reaktive Spezies wie beispielsweise Peroxid und Radikale entstehen. Da reines Wasser elektrischen Strom schlecht leitet, laufen die oben genannten Reaktionen recht langsam ab. Um die Reaktionen zu beschleunigen, werden wasserlösliche Elektrolyte, das heißt Salze, zugesetzt, um die Leitfähigkeit des Wassers zu erhöhen. Diese Elektrolyte können dann ebenfalls elektrochemische Reaktionen eingehen. Durch die Elektrolyse von beispielsweise NaCl-haltigem Wasser in einer Elektrolysezelle entstehen im Anoden- und Kathodenraum, die vorzugsweise durch eine Membran oder ein Diaphragma voneinander getrennt sind, zwei unterschiedliche Lösungen. Negativ geladene Ionen (OH⁻, Cl⁻) wandern zur Anode und reagieren dort zu Chlor (Cl₂), Sauerstoff (O₂), hypochloriger Säure (HClO) und stark verdünnter Salzsäure (HCl), sodass eine Lösung mit einem niedrigen pH-Wert und einem hohen Oxidations-Reduktionspotential entsteht. Aus Chlorid kann neben HClO auch HClO₂, ClO₂, ClO₃⁻ oder auch ClO₄⁻ entstehen. Positiv geladene Ionen (Na⁺) wandern zur Kathode und reagieren zu Natronlauge (NaOH) und Wasserstoff (H₂), sodass eine Lösung mit hohem pH-Wert und einem niedrigen Oxidations-Reduktionspotential entsteht.

Sofern die katalytisch aktive Anolytfraktion aus vollentsalztem Wasser hergestellt wurde, umfasst, insbesondere besteht sie daher aus H₂O und H₃O⁺ sowie gegebenenfalls gelöstem Sauerstoff und einer geringen Konzentration OH⁻. Sofern in einer besonders bevorzugten Ausführungsform die katalytisch aktive Anolytfraktion aus mindestens ein Salz aufweisendem Wasser hergestellt wurde, umfasst, insbesondere besteht die katalytisch aktive Anolytfraktion aus H₂O, H₃O⁺, gelöstem Sauerstoff sowie dem Anion des mindestens einen Salzes. In bevorzugter Ausführungsform weist die katalytisch aktive Anolytfraktion daher kein oder nur geringe Anteile des Kations eines Salzes auf.

In bevorzugter Ausführungsform weist eine aus vollentsalztem Wasser hergestellte katalytisch aktive Anolytfraktion einen Sauerstoffgehalt oberhalb der Sättigungskonzentration auf. Insbesondere weist eine aus vollentsalztem Wasser hergestellte katalytisch aktive Anolytfraktion einen Sauerstoffgehalt von 15 bis 25 mg/1, bevorzugt 17 bis 23 mg/1, bevorzugt 19 bis 21 mg/1, besonders bevorzugt 19,5 bis 20 mg/1 auf.

In bevorzugter Ausführungsform weist eine aus mindestens ein Salz aufweisendem Wasser hergestellte katalytisch aktive Anolytfraktion einen Sauerstoffgehalt oberhalb der Sättigungskonzentration auf. In bevorzugter Ausführungsform weist eine aus mindestens ein Salz aufweisendem Wasser hergestellte katalytisch aktive Anolytfraktion einen Sauerstoffgehalt von 15 bis 30 mg/1, bevorzugt 17 bis 27 mg/1, bevorzugt 19 bis 25 mg/1, besonders bevorzugt 20 bis 22 mg/1 auf.

Die erfindungsgemäß verwendete katalytisch aktive Anolytfraktion weist in bevorzugten Ausführungsformen einen pH-Wert auf, der oberhalb des pH-Wertes der in bekannten Verfahren als Katalysator verwendeten homogenen Säuren, insbesondere Mineralsäuren, liegt. Demgemäß liegt auch der im erfindungsgemäßen Verfahren vorherrschende pH-Wert deutlich über den in den säurekatalysierten Verfahren des Standes der Technik vorherrschenden pH-Werten. Vorteilhafterweise kann das erfindungsgemäße Verfahren ohne Zusatz eines Katalysators in homogener oder heterogener Form, insbesondere ohne Zusatz von homogenen Säuren oder Mineralsäuren, durchgeführt werden. Bevorzugt wird daher im erfindungsgemäßen Verfahren, insbesondere in den Verfahrensschritten a) bis d), insbesondere a) bis c), abgesehen von der katalytisch aktiven Anolytfraktion, keine weitere katalytisch aktive Komponente verwendet.

Erfindungsgemäß beträgt der pH-Wert der katalytisch aktiven Anolytfraktion 1,5 bis 4,5, bevorzugt 2 bis 4, bevorzugt 2,5 bis 3,5, besonders bevorzugt 2 bis 3, insbesondere 2 bis 2,5.

In einer besonders bevorzugten Ausführungsform ist die katalytisch aktive Anolytfraktion eine wässrige Lösung. Erfindungsgemäß ist bevorzugt eine einphasige Verfahrensführung vorgesehen. Bevorzugt ist eine zweiphasige Verfahrensführung ausgeschlossen. Bevorzugt ist keine Phasentrennung, insbesondere keine induzierte Phasentrennung, vorgesehen.

In besonders bevorzugter Ausführungsform ist vorgesehen, dass im erfindungsgemäßen Verfahren, insbesondere in den Verfahrensschritten a) bis d), insbesondere a) bis c), kein organisches Lösungsmittel eingesetzt wird. Insbesondere wird in den Verfahrensschritten a) bis d), insbesondere a) bis c), kein organisches Lösungsmittel eingesetzt, das mit Wasser mischbar oder mit Wasser nicht mischbar ist.

Insbesondere finden die Verfahrensschritte a) bis d) in wässriger Lösung statt.

Neben der katalytisch aktiven Anolytfraktion wird in Schritt a) des erfindungsgemäßen Verfahrens eine Fructose-haltige Komponente bereitgestellt. Diese ist bevorzugt eine feste Fructose-haltige Komponente, insbesondere Fructose, oder eine flüssige Fructose-haltige Komponente, insbesondere ein Fructosesirup, ein Fructose/Glucose-Sirup oder eine Fructoselösung, insbesondere eine wässrige Fructoselösung. Die Fructose-haltige Komponente wird daher vorliegend auch als Fructose-haltige Ausgangslösung bezeichnet. Erfindungsgemäß kann die Fructose-haltige Komponente auch aus Saccharose oder Stärke gewonnen werden oder aus Biomasse gewonnene Glucose kann zu Fructose isomerisiert werden. Bevorzugt weist die Fructose-haltige Komponente einen Trockensubstanzgehalt (TS) von 40 bis 100 Gew.-%, bevorzugt 50 bis 90 Gew.-%, bevorzugt 60 bis 85 Gew.-% Fructose auf.

In einer bevorzugten Ausführungsform der vorliegenden Erfindung werden die in Schritt a) bereitgestellten Komponenten in Schritt b) zum Erhalten einer Reaktionslösung mit einem Kohlenhydratgehalt von 5 Gew.-% bis 50 Gew.-% (Trockensubstanz, im folgenden auch TS, Kohlenhydrat in Bezug auf Gesamtgewicht Reaktionslösung) vermischt und gemäß Verfahrensschritt c) umgesetzt. Besonders bevorzugt beträgt der Kohlenhydratgehalt der Reaktionslösung in Schritt b) 10 Gew.-% bis 45 Gew.-%, bevorzugt 15 Gew.-% bis 40 Gew.-%, bevorzugt 25 Gew.-% bis 35 Gew.-%, bevorzugt 20 Gew.-%, 30 Gew.-% oder 40 Gew.-% (jeweils TS Kohlenhydrat in Bezug auf Gesamtgewicht Reaktionslösung).

In einer bevorzugten Ausführungsform des vorliegenden Verfahrens beträgt der Fructosegehalt der im Verfahrensschritt b) erhaltenen Reaktionslösung 40 Gew.-% bis 100 Gew.-%, bevorzugt 70 Gew.-% bis 100 Gew.%, bevorzugt 80 Gew.-% bis 100 Gew.%, bevorzugt 90 Gew.-% bis 100 Gew.%, bevorzugt 95 Gew.-% bis 100 Gew.%, bevorzugt 40 Gew.-% bis 99 Gew.-%, bevorzugt 45 Gew.-% bis 99 Gew.-%, bevorzugt 50 Gew.-% bis 95 Gew.-%, bevorzugt 45 Gew.-% bis 90 Gew.-%, bevorzugt 55 Gew.-% bis 85 Gew.-% (jeweils TS Fructose in Bezug auf die Trockensubstanz des Kohlenhydratanteils, das heißt alle in der Reaktionslösung vorhandenen Kohlenhydrate).

In einer besonders bevorzugten Ausführungsform der vorliegenden Erfindung werden die in Schritt a) bereitgestellten Komponenten in Schritt b) zum Erhalten einer Reaktionslösung mit einem Kohlenhydratgehalt von 5 Gew.-% bis 50 Gew.-%, bevorzugt 10 Gew.-% bis 45 Gew.-%, bevorzugt 15 Gew.-% bis 40 Gew.-%, bevorzugt 25 Gew.-% bis 35 Gew.-%, bevorzugt 20 Gew.-%, 30 Gew.-% oder 40 Gew.-%, (jeweils TS Kohlenhydrat in Bezug auf Gesamtgewicht Reaktionslösung) und einem Fructosegehalt von 40 Gew.-% bis 100 Gew.-%, bevorzugt 70 Gew.-% bis 100 Gew.%, bevorzugt 80 Gew.-% bis 100 Gew.%, bevorzugt 90 Gew.-% bis 100 Gew.%, bevorzugt 95 Gew.-% bis 100 Gew.%, bevorzugt 40 Gew.-% bis 99 Gew.-%, bevorzugt 45 Gew.-% bis 99 Gew.-%, bevorzugt 50 Gew.-% bis 95 Gew.-%, bevorzugt 45 Gew.-% bis 90 Gew.-%, bevorzugt 55 Gew.-% bis 85 Gew.-% (jeweils TS Fructose in Bezug auf die Trockensubstanz des Kohlenhydratanteils, das heißt alle in der Reaktionslösung vorhandenen Kohlenhydrate) vermischt und gemäß Verfahrensschritt c) umgesetzt.

In einer bevorzugten Ausführungsform der vorliegenden Erfindung beträgt das Verhältnis von Kohlenhydratgehalt (Trockensubstanz) der Fructose-haltigen Komponente zu katalytisch aktiver Anolytfraktion (Gesamtgewicht) in der Reaktionslösung 0,01 bis 2,5, bevorzugt 0,02 bis 2,0, bevorzugt 0,05 bis 1,5, bevorzugt 0,1 bis 1,0, bevorzugt 0,2 bis 0,9, besonders bevorzugt 0,3 bis 0,8.

In einer bevorzugten Ausführungsform der vorliegenden Erfindung beträgt das Verhältnis von Kohlenhydratgehalt (Trockensubstanz) der Fructose-haltigen Komponente zu katalytisch aktiver Anolytfraktion (Gesamtgewicht) in der Reaktionslösung 0,01 bis 2,5, bevorzugt 0,02 bis 2,0, bevorzugt 0,05 bis 1,5, bevorzugt 0,1 bis 1,0, bevorzugt 0,2 bis 0,9, besonders bevorzugt 0,3 bis 0,8 und der Kohlenhydratgehalt der Reaktionslösung insgesamt 5 Gew.-% bis 50 Gew-%, bevorzugt 10 Gew.-% bis 45 Gew.-%, bevorzugt 15 Gew.-% bis 40 Gew.-%, bevorzugt 25 Gew.-% bis 35 Gew.-%, bevorzugt 20 Gew.-%, 30 Gew.-% oder 40 Gew.-% (jeweils TS Kohlenhydrat in Bezug auf Gesamtgewicht Reaktionslösung).

In einer bevorzugten Ausführungsform der vorliegenden Erfindung beträgt das Verhältnis von Kohlenhydratgehalt (Trockensubstanz) der Fructose-haltigen Komponente zu katalytisch aktiver Anolytfraktion (Gesamtgewicht) in der Reaktionslösung 0,01 bis 2,5, bevorzugt 0,02 bis 2,0, bevorzugt 0,05 bis 1,5, bevorzugt 0,1 bis 1,0, bevorzugt 0,2 bis 0,9, besonders bevorzugt 0,3 bis 0,8 und der Kohlenhydratgehalt der Reaktionslösung insgesamt 5 Gew.-% bis 50 Gew-%, bevorzugt 10 Gew.-% bis 45 Gew.-%, bevorzugt 15 Gew.-% bis 40 Gew.-%, bevorzugt 25 Gew.-% bis 35 Gew.-%, bevorzugt 20 Gew.-%, 30 Gew.-% oder 40 Gew.-% (jeweils TS Kohlenhydrat in Bezug auf Gesamtgewicht Reaktionslösung) enthaltend einen Fructosegehalt von 40 Gew.-% bis 100 Gew.-%, bevorzugt 70 Gew.-% bis 100 Gew.%, bevorzugt 80 Gew.-% bis 100 Gew.%, bevorzugt 90 Gew.-% bis 100 Gew.%, bevorzugt 95 Gew.-% bis 100 Gew.%, bevorzugt 40 Gew.-% bis 99 Gew.-%, bevorzugt 45 Gew.-% bis 99 Gew.-%, bevorzugt 50 Gew.-% bis 95 Gew.-%, bevorzugt 45 Gew.-% bis 90 Gew.-%, bevorzugt 55 Gew.-% bis 85 Gew.-% (jeweils TS Fructose in Bezug auf die Trockensubstanz des Kohlenhydratanteils, das heißt alle in der Reaktionslösung vorhandenen Kohlenhydrate).

In einer bevorzugten Ausführungsform der vorliegenden Erfindung beträgt das Verhältnis von Fructosegehalt (Trockensubstanz) der Fructose-haltigen Komponente zu katalytisch aktiver Anolytfraktion (Gesamtgewicht) in der Reaktionslösung 0,01 bis 2,5, bevorzugt 0,02 bis 2,0, bevorzugt 0,05 bis 1,5, bevorzugt 0,1 bis 1,0, bevorzugt 0,2 bis 0,9, besonders bevorzugt 0,3 bis 0,8.

In einer bevorzugten Ausführungsform der vorliegenden Erfindung beträgt das Verhältnis von Fructosegehalt (Trockensubstanz) der Fructose-haltigen Komponente zu katalytisch aktiver Anolytfraktion (Gesamtgewicht) in der Reaktionslösung 0,01 bis 2,5, bevorzugt 0,02 bis 2,0, bevorzugt 0,05 bis 1,5, bevorzugt 0,1 bis 1,0, bevorzugt 0,2 bis 0,9, besonders bevorzugt 0,3 bis 0,8 und der Kohlenhydratgehalt der Reaktionslösung insgesamt 5 Gew.-% bis 50 Gew-%, bevorzugt 10 Gew.-% bis 45 Gew.-%, bevorzugt 15 Gew.-% bis 40 Gew.-%, bevorzugt 25 Gew.-% bis 35 Gew.-%, bevorzugt 20 Gew.-%, 30 Gew.-% oder 40 Gew.-% (jeweils TS Kohlenhydrat in Bezug auf Gesamtgewicht Reaktionslösung).

In einer bevorzugten Ausführungsform der vorliegenden Erfindung beträgt das Verhältnis von Fructosegehalt (Trockensubstanz) der Fructose-haltigen Komponente zu katalytisch aktiver Anolytfraktion (Gesamtgewicht) in der Reaktionslösung 0,01 bis 2,5, bevorzugt 0,02 bis 2,0, bevorzugt 0,05 bis 1,5, bevorzugt 0,1 bis 1,0, bevorzugt 0,2 bis 0,9, besonders bevorzugt 0,3 bis 0,8 und der Kohlenhydratgehalt der Reaktionslösung insgesamt 5 Gew.-% bis 50 Gew-%, bevorzugt 10 Gew.-% bis 45 Gew.-%, bevorzugt 15 Gew.-% bis 40 Gew.-%, bevorzugt 25 Gew.-% bis 35 Gew.-%, bevorzugt 20 Gew.-%, 30 Gew.-% oder 40 Gew.-% (jeweils TS Kohlenhydrat in Bezug auf Gesamtgewicht Reaktionslösung) enthaltend einen Fructosegehalt von 40 Gew.-% bis 100 Gew.-%, bevorzugt 70 Gew.-% bis 100 Gew.%, bevorzugt 80 Gew.-% bis 100 Gew.%, bevorzugt 90 Gew.-% bis 100 Gew.%, bevorzugt 95 Gew.-% bis 100 Gew.%, bevorzugt 40 Gew.-% bis 99 Gew.-%, bevorzugt 45 Gew.-% bis 99 Gew.-%, bevorzugt 50 Gew.-% bis 95 Gew.-%, bevorzugt 45 Gew.-% bis 90 Gew.-%, bevorzugt 55 Gew.-% bis 85 Gew.-% (jeweils TS Fructose in Bezug auf die Trockensubstanz des Kohlenhydratanteils, das heißt alle in der Reaktionslösung vorhandenen Kohlenhydrate).

In einer bevorzugten Ausführungsform der vorliegenden Erfindung beträgt das Verhältnis von katalytisch aktiver Anolytfraktion (Gesamtgewicht) zu Kohlenhydratgehalt (Trockensubstanz) der Fructose-haltigen Komponente in der Reaktionslösung 0,4 bis 100, bevorzugt 0,5 bis 50, bevorzugt 0,7 bis 20, bevorzugt 1,0 bis 10, besonders bevorzugt 1,1 bis 5, bevorzugt 1,25 bis 3,3.

In einer bevorzugten Ausführungsform der vorliegenden Erfindung beträgt das Verhältnis von katalytisch aktiver Anolytfraktion (Gesamtgewicht) zu Fructosegehalt (Trockensubstanz) der Fructose-haltigen Komponente in der Reaktionslösung 0,4 bis 100, bevorzugt 0,5 bis 50, bevorzugt 0,7 bis 20, bevorzugt 1,0 bis 10, besonders bevorzugt 1,1 bis 5, bevorzugt 1,25 bis 3,3.

Erfindungsgemäß bevorzugt beträgt die Konzentration an Anionen der katalytisch aktiven Anolytfraktion in der in Verfahrensschritt b) erhaltenen Reaktionslösung 1 × 10⁻⁵ bis 0,5 mol/l, bevorzugt 1,5 × 10⁻⁵ bis 0,45 mol/1, bevorzugt 1 × 10⁻⁴ bis 0,4 mol/1, bevorzugt 1 × 10⁻³ bis 0,35 mol/1, besonders bevorzugt 0,01 bis 0,3 mol/l.

In besonders bevorzugter Ausführungsform findet das Vermischen, also Schritt b) des erfindungsgemäßen Verfahrens, der für die Herstellung der Reaktionslösung eingesetzten Komponenten, das heißt insbesondere der Fructose-haltigen Komponente und der katalytisch aktiven Anolytfraktion in einer Mischvorrichtung und/oder einer Leitung statt. Die Mischvorrichtung beziehungsweise die Leitung und das Reaktorsystem, in dem die Umsetzung, das heißt Schritt c), des vorliegenden Verfahrens stattfindet, können räumlich getrennte Baueinheiten darstellen, die durch mindestens eine Leitung miteinander verbunden sind, sie können auch getrennte, aber integrale Bestandteile einer Vorrichtung darstellen. Bevorzugt wird die Reaktionslösung mithilfe einer Pumpe, insbesondere einer Hochdruckpumpe, in das Reaktorsystem eingebracht.

In einer bevorzugten Ausführungsform der vorliegenden Erfindung wird die in Schritt a) bereitgestellte Fructose-haltige Komponente, die Anolytfraktion oder beide vor Schritt b) auf eine Temperatur von 90 °C bis 200 °C eingestellt. Bevorzugt wird also vor Schritt b) mindestens eine, vorzugsweise alle, der in Schritt a) bereitgestellten Komponenten, das heißt die Fructose-haltige Komponente und die katalytisch aktive Anolytfraktion getrennt voneinander auf eine Temperatur von 90 °C bis 200°C, bevorzugt 100 °C bis 175 °C, bevorzugt 150 °C bis 175 °C vorgeheizt. In einer bevorzugten Ausführungsform der vorliegenden Erfindung wird vor Schritt b) mindestens eine, vorzugsweise alle, der in Schritt a) bereitgestellten Komponenten auf eine Temperatur von 120 °C bis 180 °C, bevorzugt 130 °C bis 180 °C, bevorzugt 140 °C bis 180 °C vorgeheizt. Insbesondere wird vor Schritt b) mindestens eine, vorzugsweise alle, der in Schritt a) bereitgestellten Komponenten getrennt voneinander auf eine Temperatur von 160 °C, 165 °C, 170°C oder 175 °C vorgeheizt.

In einer alternativen bevorzugten Ausführungsform der vorliegenden Erfindung wird die in Schritt b) erhaltene Reaktionslösung auf eine Temperatur von 90 °C bis 200 °C eingestellt. Bevorzugt wird also die in Schritt b) durch das Vermischen der in Schritt a) bereitgestellten Komponenten erhaltene Reaktionslösung, bevorzugt nach Schritt b) und vor Schritt c), auf eine Temperatur von 90 °C bis 200 °C, bevorzugt 100 °C bis 175 °C, bevorzugt 150 °C bis 175 °C erhitzt. Bevorzugt wird die in Schritt b) erhaltene Reaktionslösung, bevorzugt nach Schritt b) und vor Schritt c), auf eine Temperatur von 120 °C bis 180 °C, bevorzugt 130 °C bis 180 °C, bevorzugt 140 °C bis 180 °C erhitzt. Insbesondere wird die in Schritt b) erhaltene Reaktionslösung auf eine Temperatur von 160 °C, 165 °C, 170°C oder 175 °C erhitzt.

In besonders bevorzugter Ausführungsform wird der anschießende Schritt c) des vorliegenden Verfahrens, also das Umsetzen der in der Reaktionslösung vorhandenen Fructose zu HMF, bei einer Temperatur von 90 bis 200 °C, insbesondere 120 bis 195 °C, insbesondere 140 bis 190 °C, insbesondere 150 bis 180 °C, insbesondere 160 bis 175 °C, insbesondere 165 bis 170 °C, insbesondere 165 bis 175 °C, insbesondere 170 bis 175 °C, insbesondere 160 bis 165 °C, insbesondere 165 °C, insbesondere 170 °C, insbesondere 175 °C durchgeführt.

Erfindungsgemäß beträgt die zur Durchführung des erfindungsgemäßen Verfahrens verwendete Temperatur in bevorzugter Ausführungsform zu jedem Zeitpunkt höchstens 200 °C, bevorzugt höchstens 175 °C, insbesondere höchstens 165 °C.

In einer bevorzugten Ausführungsform der vorliegenden Erfindung erfolgt die Umsetzung der in der Reaktionslösung enthaltenen Fructose zu HMF in Schritt c) in einem Zeitraum von 0,1 bis 20 min, insbesondere 0,1 bis 15 min, insbesondere 8 bis 13 min, insbesondere 4 bis 10 min, insbesondere 8 bis 10 min, bevorzugt 0,1 bis 8 min, bevorzugt 0,2 bis 7 min, bevorzugt 0,5 bis 5 min, bevorzugt 1 bis 4 min, bevorzugt 5 bis 6 min. Bevorzugt erfolgt die Umsetzung der Fructose zu HMF in Schritt c) in einem Zeitraum von höchstens 10 min, bevorzugt höchstens 9 min, bevorzugt höchstens 8 min, bevorzugt höchstens 7 min, bevorzugt höchstens 6 min, bevorzugt höchstens 5 min, bevorzugt höchstens 4 Minuten.

In bevorzugter Ausführungsform sieht die Erfindung vor, dass in Schritt c) ein Fructoseumsatz von 1 mol-% bis 50 mol-% erreicht wird. In einer bevorzugten Ausführungsform erfolgt die Umsetzung der Fructose zu HMF in Schritt c) unter Einstellung eines Fructose-Umsatzes von 1 mol-% bis 50 mol-%, bevorzugt 5 mol-% bis 40 mol-%, bevorzugt 10 mol-% bis 30 mol-%, bevorzugt 15 mol-% bis 25 mol-%, bevorzugt 20 mol-% bis 25 mol-%. Bevorzugt erfolgt die Umsetzung der Fructose zu HMF in Schritt c) unter Einstellung eines Fructose-Umsatzes von höchstens 50 mol-%, bevorzugt höchstens 40 mol-%, bevorzugt höchstens 30 mol-%, bevorzugt höchstens 25 mol-%, bevorzugt höchstens 20 mol-%. Dies erfolgt erfindungsgemäß bei einer Temperatur von 90 °C bis 200 °C.

Im Zusammenhang mit der vorliegenden Erfindung wird unter "Einstellung eines FructoseUmsatzes" verstanden, dass die für die Umsetzung von Fructose zu HMF verwendeten Reaktionsparameter, insbesondere die Reaktionstemperatur und die Reaktionsdauer im Reaktor so gewählt werden, dass es lediglich zu einer limitierten Umsetzung der Fructose von maximal 50 mol-% kommt, wodurch eine hohe HMF-Selektivität und damit verbunden eine geringe Nebenproduktbildung erreicht werden kann.

Bevorzugt ist es also möglich, im Rahmen der vorgegebenen Parameter gezielt definierte Fructoseumsätze bereitzustellen, insbesondere durch Verwendung der erfindungsgemäß bevorzugten Reaktionstemperatur, gegebenenfalls in bevorzugter Ausführungsform auch der Reaktionszeit, für Schritt c). Anhand dieser Parameter kann auch eine erfindungsgemäß bevorzugte HMF-Selektivität eingestellt werden. In erfindungsgemäß bevorzugter Weise kann die Einstellung des gewünschten Fructoseumsatzes, und gegebenenfalls der HMF-Selektivität, durch Probeentnahme während des Verfahrens, Analyse der Probe und anschließender Berechnung der zum Erreichen der gewünschten Fructoseumsatzwerte und der gegebenenfalls gewünschten HMF-Selektivität aufrecht zu erhaltenden oder anzupassenden Parameter erfolgen. In einer besonders bevorzugten Ausführungsform erfolgt die Umsetzung der in der Fructose-haltigen Komponente enthaltenen Fructose in Schritt c) bei einer Temperatur von 90 bis 200 °C, bevorzugt 150 bis 190 °C, insbesondere 160 °C, 165 °C, 170 °C oder 175°C für einen Zeitraum von 4 bis 7 min, bevorzugt 5 bis 6 min, insbesondere 5,6 Minuten. Dies führt bevorzugt zu einem Fructoseumsatz von 1 bis 50 mol-%.

In einer bevorzugten Ausführungsform der vorliegenden Erfindung wird das Verfahren so eingestellt, dass in Schritt c) eine HMF-Selektivität von 60 mol-% bis 100 mol-%, bevorzugt 65 mol-% bis 100 mol-%, bevorzugt 70 mol-% bis 100 mol-%, bevorzugt 75 mol-% bis 100 mol-%, bevorzugt 80 mol-% bis 100 mol-%, bevorzugt 85 mol-% bis 100 mol-%, bevorzugt 90 mol-% bis 100 mol-% erhalten wird. Bevorzugt beträgt die HMF-Selektivität in Schritt c) mindestens 60 mol-%, bevorzugt mindestens 65 mol-%, bevorzugt mindestens 70 mol-%, bevorzugt mindestens 75 mol-%, bevorzugt mindestens 80 mol-%, bevorzugt mindestens 85 mol-%, bevorzugt mindestens 90 mol-%, bevorzugt mindestens 95 mol-%.

In einer bevorzugten Ausführungsform der vorliegenden Erfindung wird das Verfahren so eingestellt, dass in Schritt c) eine HMF-Selektivität von 60 mol-% bis 100 mol-%, bevorzugt 65 mol-% bis 100 mol-%, bevorzugt 70 mol-% bis 100 mol-%, bevorzugt 75 mol-% bis 100 mol-%, bevorzugt 80 mol-% bis 100 mol-%, bevorzugt 85 mol-% bis 100 mol-%, bevorzugt 90 mol-% bis 100 mol-%, bevorzugt mindestens 60 mol-%, bevorzugt mindestens 65 mol-%, bevorzugt mindestens 70 mol-%, bevorzugt mindestens 75 mol-%, bevorzugt mindestens 80 mol-%, bevorzugt mindestens 85 mol-%, bevorzugt mindestens 90 mol-%, bevorzugt mindestens 95 mol-% und ein Fructoseumsatz von 1 mol-% bis 50 mol-%, bevorzugt 5 mol-% bis 40 mol-%, bevorzugt 10 mol-% bis 30 mol-%, bevorzugt 15 mol-% bis 25 mol-%, bevorzugt 20 mol-% bis 25 mol-%, bevorzugt höchstens 50 mol-%, bevorzugt höchstens 40 mol-%, bevorzugt höchstens 30 mol-%, bevorzugt höchstens 25 mol-%, bevorzugt höchstens 20 mol-% erhalten wird.

In einer besonders bevorzugten Ausführungsform der vorliegenden Erfindung wird das Verfahren so eingestellt, dass in Schritt c) eine HMF-Selektivität von 60 mol-% bis 100 mol-%, bevorzugt 65 mol-% bis 100 mol-%, bevorzugt 70 mol-% bis 100 mol-%, bevorzugt 75 mol-% bis 100 mol-%, bevorzugt 80 mol-% bis 100 mol-%, bevorzugt 85 mol-% bis 100 mol-%, bevorzugt 90 mol-% bis 100 mol-%, bevorzugt mindestens 60 mol-%, bevorzugt mindestens 65 mol-%, bevorzugt mindestens 70 mol-%, bevorzugt mindestens 75 mol-%, bevorzugt mindestens 80 mol-%, bevorzugt mindestens 85 mol-%, bevorzugt mindestens 90 mol-%, bevorzugt mindestens 95 mol-% und ein Fructoseumsatz von 1 mol-% bis 50 mol-%, bevorzugt 5 mol-% bis 40 mol-%, bevorzugt 10 mol-% bis 30 mol-%, bevorzugt 15 mol-% bis 25 mol-%, bevorzugt 20 mol-% bis 25 mol-%, bevorzugt höchstens 50 mol-%, bevorzugt höchstens 40 mol-%, bevorzugt höchstens 30 mol-%, bevorzugt höchstens 25 mol-%, bevorzugt höchstens 20 mol-% erhalten wird, wobei dies mithilfe einer Temperatur von 90 bis 200 °C, insbesondere 140 bis 190 °C, insbesondere 150 bis 180 °C, insbesondere 160 bis 175 °C, insbesondere 165 bis 170 °C, insbesondere 165 bis 175 °C, insbesondere 170 bis 175 °C, insbesondere 160 bis 165 °C, insbesondere 165 °C, insbesondere 170 °C, insbesondere 175 °C und innerhalb eines Zeitraums von 0,1 bis 20 min, insbesondere 0,1 bis 15 min, insbesondere 8 bis 13 min, insbesondere 4 bis 10 min, insbesondere 8 bis 10 min, bevorzugt 0,1 bis 8 min, bevorzugt 0,2 bis 7 min, bevorzugt 0,5 bis 5 min, bevorzugt 1 bis 4 min, bevorzugt 5 bis 6 min erreicht wird.

Im Zusammenhang mit der vorliegenden Erfindung wird die HMF-Selektivität auf den umgesetzten Fructoseanteil bezogen, wobei Anteile anderer Kohlenhydrate, insbesondere Glucose unberücksichtigt bleiben.

In einer bevorzugten Ausführungsform der vorliegenden Erfindung liegt die HMF-Ausbeute bei 3 bis 50 mol-%, bevorzugt 5 bis 45 mol-%, bevorzugt 10 bis 40 mol-%, bevorzugt 15 bis 35 mol-%, besonders bevorzugt bei 20 bis 30 mol-%.

In einer bevorzugten Ausführungsform der vorliegenden Erfindung wird in Schritt c) der Druck zum Umsetzen der in der Reaktionslösung vorhandenen Fructose zu HMF so eingestellt, dass ein Sieden der Reaktionslösung und damit das Auftreten von Dampfblasen vermieden wird. Bevorzugt liegt der Druck zum Umsetzen der in der Reaktionslösung vorhandenen Fructose zu HMF im Reaktorsystem bei 0,1 bis 2 MPa, bevorzugt 0,2 bis 1,5 MPa, besonders bevorzugt 1 MPa.

Erfindungsgemäß ist vorgesehen, dass die in der Reaktionslösung vorhandene Fructose in Schritt c) unter Einstellung verschiedener Parameter wie Temperatur, Reaktionszeit und/oder Druck zu HMF umgesetzt wird und in Schritt d) eine flüssige HMF-haltige Produktmischung erhalten wird. Bevorzugt wird das Verfahren also so geführt, dass es durch Einstellung der Temperatur, und bevorzugt auch der Reaktionszeit, zielgerichtet zu einer limitierten Umsetzung der Fructose von 1 mol-% bis 50 mol-% kommt, wodurch eine überraschend hohe HMF-Selektivität, bevorzugt von 60 mol-% bis 100 mol-%, erreicht werden kann.

In einer besonders bevorzugten Ausführungsform sieht das erfindungsgemäß vorgesehene Umsetzen der in der Reaktionslösung vorhandenen Fructose zu HMF und das Erhalten von HMF gemäß der Verfahrensschritte c) und d) ein einstufiges Verfahren vor. Insbesondere ist die erfindungsgemäße Verfahrensweise gemäß der Verfahrensschritte c) und d) bevorzugt keine zweistufige Verfahrensweise.

In einer bevorzugten Ausführungsform umfasst das vorliegende Verfahren weiterhin Schritt:
e) Abkühlen der in Schritt d) erhaltenen flüssigen HMF-Produktmischung auf eine Temperatur von 20 °C bis 80 °C, bevorzugt 25 °C bis 70 °C, bevorzugt 30 °C bis 60 °C, bevorzugt 30 °C bis 55 °C, bevorzugt 30 °C bis 50 °C, bevorzugt 30 °C bis 45 °C, bevorzugt 30 °C bis 40 °C, bevorzugt 80 °C, bevorzugt 70 °C, bevorzugt 60 °C, bevorzugt 55 °C, bevorzugt 50 °C, bevorzugt 45 °C, bevorzugt 40 °C, bevorzugt 35 °C, bevorzugt 30 °C. Bevorzugt wird die flüssige HMF-Produktmischung in Schritt e) auf eine Temperatur von höchstens 75°C, bevorzugt höchstens 70 °C, bevorzugt höchstens 60 °C, bevorzugt höchstens 55 °C, bevorzugt höchstens 50 °C, bevorzugt höchstens 45 °C, bevorzugt höchstens 40 °C, bevorzugt höchstens 35 °C abgekühlt. Dies kann erfindungsgemäß ein- oder zweistufig erfolgen.

In einer bevorzugten Ausführungsform der vorliegenden Erfindung wird die Temperatur der flüssigen HMF-Produktmischung in Schritt e) in einem Zeitraum von 0,1 bis 10 min, bevorzugt 0,1 bis 9 min, bevorzugt 0,1 bis 8 min, bevorzugt 0,2 bis 7 min, bevorzugt 0,2 bis 6 min, bevorzugt 0,5 bis 5 min, bevorzugt 0,5 bis 4 min, bevorzugt 0,5 bis 3 min eingestellt, beziehungsweise abgekühlt. Bevorzugt wird die Temperatur der Produktmischung in Schritt e) in höchstens 10 min, bevorzugt höchstens 9 min, bevorzugt höchstens 8 min, bevorzugt höchstens 7 min, bevorzugt höchstens 6 min, bevorzugt höchstens 5 min, bevorzugt höchstens 4 min, bevorzugt höchstens 3 min, bevorzugt höchstens 2 min, bevorzugt höchstens 1 min, bevorzugt höchstens 0,5 min eingestellt, beziehungsweise abgekühlt.

Somit wird die in Schritt d) erhaltene HMF-haltige Produktmischung nach Erreichen des limitierten Fructose-Umsatzes von maximal 50 mol-% in Schritt e) auf eine Temperatur von 20 °C bis 80 °C abgekühlt. Dies verhindert vorteilhafterweise weitestgehend die Bildung unerwünschter Nebenprodukte sowie die Zersetzung des gebildeten HMF.

Das erfindungsgemäße Verfahren zur Herstellung von HMF wird bevorzugt in einem geeigneten Reaktorsystem durchgeführt. Erfindungsgemäß bevorzugt handelt es sich dabei um ein kontinuierliches Reaktorsystem.

In besonders bevorzugter Ausführungsform wird das eingesetzte kontinuierliche Reaktorsystem als Rohrreaktorsystem ausgeführt. Ein derartiges kontinuierliches Reaktorsystem ist ein dem Fachmann bekanntes Reaktorsystem. In besonders bevorzugter Ausführungsform kann auch ein kontinuierliches Reaktorsystem, insbesondere ein Konti-System, mit geringer Rückvermischung eingesetzt werden. In besonders bevorzugter Ausführungsform kann als kontinuierliches Reaktorsystem ein plug-flow-Reaktor (PFR) eingesetzt werden. In bevorzugter Ausführungsform kann das kontinuierliche Reaktorsystem auch als Strömungsrohr, Rührkessel oder Rührkesselkaskade ausgeführt sein. Im Zusammenhang mit der vorliegenden Erfindung wird unter einem plug-flow-Reaktor (PFR) ein sogenanntes ideales Strömungsrohr (IR) verstanden, das heißt ein rohrförmiger Reaktor, in welchem eine Pfropfenströmung vorliegt. Ein derartiger Reaktor ist insbesondere auch dadurch ausgezeichnet, dass keine Vermischung, Rückströmung oder Verwirbelung der durchgeführten Reaktionslösung stattfindet, sondern vielmehr eine gleichmäßige Durchströmung unter parallel stattfindender Stoffumwandlung stattfindet. Der plug-flow-Reaktor stellt insbesondere sicher, dass jeder in den plug-flow-Reaktor eingespeiste Stoff, insbesondere jede eingespeiste Komponente, unter gleichen Bedingungen kontinuierlich umgesetzt wird, das heißt alle Komponenten dem Umsetzungsprozess für die gleiche Zeitdauer ausgesetzt werden.

In einer bevorzugten Ausführungsform umfasst das vorliegende Verfahren optional weiterhin den Schritt:
f) Filtration, Entfärbung und/oder Reinigung der flüssigen HMF-Produktmischung.

Das heißt, in einer weiteren bevorzugten Ausführungsform findet eine Filtration der HMF-Produktmischung, bevorzugt über einen geeigneten Filter oder ein geeignetes Filtersystem, und eine Entfärbung und/oder Reinigung der Produktmischung, bevorzugt eine Entfärbung und/oder Reinigung über Aktivkohle, statt. Bevorzugt erfolgt eine Filtration der Produktmischung über einen geeigneten Filter oder ein geeignetes Filtersystem und eine Entfärbung und/oder Reinigung der Produktmischung zum Beispiel über Aktivkohle nach Schritt e). Bevorzugt erfolgt eine Filtration der Produktmischung über einen geeigneten Filter oder ein geeignetes Filtersystem und eine Entfärbung und/oder Reinigung der Produktmischung zum Beispiel über Aktivkohle vor Schritt g) oder h). In besonders bevorzugter Ausführungsform kann nach Verfahrensschritt e) und/oder Verfahrensschritt g) in beliebiger Reihenfolge eine Filtration über einen geeigneten Filter oder ein geeignetes Filtersystem, eine Entfärbung und/oder Reinigung der Produktmischung, insbesondere über Aktivkohle und gegebenenfalls nach Schritt g) eine nochmalige Filtration über einen geeigneten Filter oder ein geeignetes Filtersystem durchgeführt werden. In besonders bevorzugter Ausführungsform wird nach Schritt e) und/oder Verfahrensschritt g) zunächst eine Filtration über einen geeigneten Filter oder ein geeignetes Filtersystem, dann eine Entfärbung und/oder Reinigung, insbesondere über Aktivkohle und gegebenenfalls nach Schritt g) eine nochmalige Filtration über einen geeigneten Filter oder ein geeignetes Filtersystem in dieser Reihenfolge durchgeführt. Erfindungsgemäß bevorzugt wird für die Filtration ein Metallsinterfilter verwendet.

Bevorzugt werden durch die Filtration der Produktmischung über einen geeigneten Filter oder ein geeignetes Filtersystem und Entfärbung und/oder Reinigung über zum Beispiel Aktivkohle unerwünschte Nebenprodukte, insbesondere lösliche und unlösliche Huminstoffe, aus der Produktmischung entfernt.

In einer bevorzugten Ausführungsform der vorliegenden Erfindung weist die in Schritt e) oder ggf. Schritt f) erhaltene Produktmischung einen Trockensubstanzgehalt von 5 bis 50 Gew.-%, bevorzugt 10 bis 40 Gew.-%, bevorzugt mindestens 5 Gew.-%, bevorzugt mindestens 10 Gew.%, bevorzugt höchstens 50 Gew.-%, bevorzugt höchstens 40 Gew.-% auf.

Sollte der Trockensubstanzgehalt der in Schritt e) oder gegebenenfalls f) erhaltenen Produktmischung zu niedrig sein, kann es erfindungsgemäß vorgesehen sein, dass das vorliegende Verfahren optional weiterhin den Schritt umfasst:
g) Einstellen der flüssigen HMF-Produktmischung auf einen Trockensubstanzgehalt von 20 bis 70 Gew.-%, bevorzugt 25 bis 60 Gew.-%, bevorzugt 25 bis 50 Gew.-%, bevorzugt 30 bis 45 Gew.-%, bevorzugt 30 bis 40 Gew.-%.

In einer weiteren bevorzugten Ausführungsform wird die in Schritt e) oder gegebenenfalls f) erhaltene Produktmischung auf einen Trockensubstanzgehalt von 20 bis 70 Gew.-%, bevorzugt mindestens 20 Gew.-%, bevorzugt mindestens 30 Gew.-%, bevorzugt mindestens 40 Gew.-%, bevorzugt mindestens 50 Gew.-%, bevorzugt höchstens 70 Gew.-%, bevorzugt höchstens 60 Gew.-%, bevorzugt höchstens 50 Gew.-% eingestellt.

In einer bevorzugten Ausführungsform umfasst das vorliegende Verfahren weiterhin die Schritte:
h) Aufreinigen der flüssigen HMF-Produktmischung mittels Chromatographie, Ultraund/oder Nanofiltration, Extraktion mit einem geeigneten Extraktionsmittel, Adsorption an ein geeignetes Material und anschließender gezielter Desorption und/oder Elektrodialyse zur Abtrennung mindestens einer HMF-Fraktion, und
i) Erhalten mindestens einer HMF-Fraktion.

Das heißt, bevorzugt wird durch die Anwendung mindestens eines der genannten Aufreinigungsprozesse mindestens eine HMF-Fraktion aus der flüssigen HMF-haltigen Produktmischung abgetrennt, sodass lediglich andere in der Produktmischung enthaltene Komponenten wie beispielsweise nicht umgesetzte Fructose, Glucose oder Nebenprodukte wie organische Säuren und Humine übrigbleiben. Es kann erfindungsgemäß auch vorgesehen sein eine Kombination von mindestens zwei oder mehr der genannten Aufreinigungsprozesse für die Abtrennung mindestens einer HMF-Fraktion und/oder gegebenenfalls anderen Fraktionen enthaltend eine oder mehrere andere Komponenten der Produktmischung, anzuwenden.

In einer alternativen bevorzugten Ausführungsform umfasst das vorliegende Verfahren weiterhin die Schritte:
h) Aufreinigen der flüssigen HMF-Produktmischung mittels Chromatographie, Ultraund/oder Nanofiltration, Extraktion mit einem geeigneten Extraktionsmittel, Adsorption an ein geeignetes Material und anschließender gezielter Desorption und/oder Elektrodialyse zur Abtrennung mindestens einer Fraktion ausgewählt aus der Gruppe bestehend aus einer HMF-Fraktion, einer Glucose-Fraktion, einer Fructose-Fraktion und einer organischen Säuren-Fraktion, und
i) Erhalten mindestens einer Fraktion ausgewählt aus der Gruppe bestehend aus einer HMF-Fraktion, einer Glucose-Fraktion, einer Fructose-Fraktion und einer organischen Säuren-Fraktion.

Es kann weiterhin vorgesehen sein mindestens eine der in Schritt i) erhaltenen Fraktionen mithilfe eines Aufreinigungsprozesses ausgewählt aus der Gruppe bestehend aus Chromatographie, Ultra- und/oder Nanofiltration, Extraktion mit einem geeigneten Extraktionsmittel, Adsorption an ein geeignetes Material und anschließender gezielter Desorption und/oder Elektrodialyse weiterzubehandeln.

Einer der im erfindungsgemäßen Verfahren vorgesehenen Aufreinigungsprozesse ist die Ultraund/oder Nanofiltration. Hierbei kann über geeignete Membranen zum einen eine Aufkonzentration der flüssigen HMF-haltigen Produktmischung erfolgen, zum anderen aber auch eine Entfernung von löslichen und/oder unlöslichen Huminen oder im Falle von Nanofiltration auch eine Abtrennung von HMF und/oder organischen Säuren aus der Produktmischung erfolgen. Bevorzugt kann durch die Ultra- und/oder Nanofiltration daher eine aufkonzentrierte Produktmischung, eine von löslichen und/oder unlöslichen Huminstoffen befreite Produktmischung, eine HMF-Fraktion und eine von HMF befreite Produktmischung, eine HMF-Fraktion und/oder eine organische Säuren Fraktion und eine von HMF und/oder organischen Säuren befreite Produktmischung, oder eine Glucose- und/oder Fructose-Fraktion und eine von Huminen und/oder Glucose und/oder Fructose befreite Produktmischung erhalten werden.

Ein weiterer im erfindungsgemäßen Verfahren vorgesehener Aufreinigungsprozess ist die Extraktion mit einem geeigneten Extraktionsmittel. Zur Extraktion von HMF aus der HMF-haltigen Produktmischung wird vorzugsweise ein Lösungsmittel verwendet, welches nicht oder kaum mit Wasser mischbar ist und welches eine ausreichend hohe Affinität zu HMF aufweist. Idealerweise ist der Siedepunkt des organischen Lösungsmittels bevorzugt relativ niedrig und der Dichteunterschied zwischen Wasser und dem Lösungsmittel ausreichend hoch sodass eine Phasentrennung erzielt werden kann. Geeignete Lösungsmittel sind bevorzugt Methylisobutylketon, Ethylacetat, Methylethylketon, Butanol, Diethylether, Methylbutylether, Isoamylalkohol, Methyltetrahydrofuran oder ähnliche. Nach dem Extraktionsschritt bleibt eine wässrige Produktmischung, die nicht umgesetzte Fructose und Glucose enthält, übrig und es wird eine organische Phase, die HMF und ggf. organische Säuren enthält, erhalten.

Ein weiterer im erfindungsgemäßen Verfahren vorgesehener Aufreinigungsprozess ist die Adsorption an ein geeignetes Material und die anschließende Desorption. Die Adsorption von HMF kann prinzipiell an jedes Material erfolgen das bevorzugt HMF aus Hexose-haltigen Lösungen adsorbiert. Bevorzugte Materialien sind Polymer-basierte Harze wie beispielsweise Divinylbenzol-Styrol-Copolymere, Adsorber-Harze, Aktivkohlen, Zeolithe, Aluminiumoxide, nicht-funktionalisierte Harze oder Kationentauscherharze. Die in Schritt e), f) oder g) erhaltene Produktmischung wird vorzugsweise kontinuierlich mit dem HMF-adsorbierenden Material in Kontakt gebracht, jedoch maximal bis zur Erschöpfung des Materials. Anschließend wird das adsorbierte HMF mit einem geeigneten Desorptionsmittel wie beispielsweise Wasser oder polaren organischen Lösungsmitteln wie Alkoholen, Ethylacetat, THF oder ähnlichen desorbiert. Aus dem organischen Lösungsmittel kann HMF dann durch geeignete Verfahren gewonnen werden.

Ein weiterer im erfindungsgemäßen Verfahren vorgesehener Aufreinigungsprozess ist die Elektrodialyse. Diese ist ein elektrochemisch getriebener Membranprozess, bei dem Ionentauschermembranen in Kombination mit einer elektrischen Potentialdifferenz genutzt werden, um ionische Spezies von ungeladenen Spezies oder Verunreinigungen in Lösung zu trennen. Im Falle des vorliegenden Prozesses kann die Elektrodialyse dazu genutzt werden, die Produktmischung von anorganischen und/oder organischen Kationen und Anionen wie beispielsweise Elektrolyte aus der Anolytfraktion, Lävulin- und Ameisensäure als Nebenprodukte zu befreien.

Ein weiterer im erfindungsgemäßen Verfahren vorgesehener Aufreinigungsprozess ist die Chromatographie. Diese wird nachstehend näher erläutert.

Alle vorstehend genannten Aufreinigungsprozesse können einzeln oder auch in Kombination miteinander angewendet werden.

Besonders bevorzugt wird in Schritt h) das in der Produktmischung enthaltene HMF unter Verwendung eines chromatographischen Verfahrens, insbesondere mittels Chromatographie an Ionenaustauscherharzen, insbesondere Kationenaustauscherharzen, insbesondere mittels einoder mehrstufiger Chromatographie an Ionenaustauscherharzen, insbesondere Kationenaustauscherharzen, von den weiteren Komponenten der Produktmischung abgetrennt.

In einer besonders bevorzugten Ausführungsform der vorliegenden Erfindung ist die Chromatographie, insbesondere Chromatographie an Ionenaustauscherharzen, insbesondere Chromatographie an Kationenaustauscherharzen, eine Ionenaustauschchromatographie, insbesondere eine Kationenaustauschchromatographie.

In einer bevorzugten Ausführungsform der vorliegenden Erfindung wird die flüssige HMF-Produktmischung in Schritt h) mittels Chromatographie in mindestens vier Fraktionen, umfassend eine HMF-Fraktion, eine Glucose-Fraktion, eine Fructose-Fraktion und eine organische Säuren Fraktion aufgetrennt und in Schritt i) mindestens eine HMF-Fraktion, eine Glucose-Fraktion, eine Fructose-Fraktion und eine organische Säuren Fraktion erhalten.

Besonders bevorzugt erfolgt die Aufreinigung der in Schritt e), gegebenenfalls f) oder gegebenenfalls g) erhaltenen Produktmischung gemäß Schritt h) mittels Chromatographie kontinuierlich. Unter einer kontinuierlichen Chromatographie wird bevorzugt auch eine simulierte Gegenstromchromatographie, wie zum Beispiel die Simulated Moving Bed Chromatography (SMB), verstanden.

Kontinuierliche Chromatographieverfahren sind dem Fachmann allgemein bekannt. Beispielsweise zeigt die US 2011 /0137084 A1 die Funktionsweise der SMB Verfahrens. Weitere geeignete Chromatographieverfahren sind in A. Rajendran et al.; J. Chromatogr. A 1216 (2009), Seiten 709 bis 738 offenbart.

Simulated Moving Bed (SMB) Systeme beziehungsweise Weiterentwicklungen des SMB-Systems, wie zum Beispiel Sequential SMB (SSMB), Intermittent/Improved SMB (ISMB) oder New MCI (NMCI) erlauben in vorteilhafter Weise die Trennung und die Gewinnung der vier beschriebenen Fraktionen in kontinuierlicher Betriebsweise.

In einer bevorzugten Ausführungsform der vorliegenden Erfindung ist die Chromatographie, insbesondere Chromatographie an Ionenaustauscherharzen, in Schritt h) ein Simulated Moving Bed-Verfahren (SMB), ein Sequential Simulated Moving Bed-Verfahren (SSMB) oder ein Improved Simulated Moving Bed-Verfahren beziehungsweise Intermittent Simulated Moving Bed-Verfahren (ISMB). Bevorzugt ist die Chromatographie, insbesondere Chromatographie an Ionenaustauscherharzen, in Schritt h) ein Simulated Moving Bed-Verfahren (SMB), ein Sequential Simulated Moving Bed-Verfahren (SSMB), ein Improved Simulated Moving Bed-Verfahren (ISMB) oder ein New MCI-Verfahren (NMCI). Durch die Verwendung eines Simulated Moving Bed-Verfahrens (SMB), eines Sequential Simulated Moving Bed-Verfahrens (SSMB), eines Improved Simulated Moving Bed-Verfahrens (ISMB) oder eines New MCI-Verfahrens (NMCI) in Schritt h) ist es vorteilhafterweise möglich, die Aufreinigung der in Schritt e), f) oder g) erhaltenen Produktmischung zur Abtrennung einer HMF-Fraktion, einer Glucose-Fraktion, einer Fructose-Fraktion und einer organische Säuren-Fraktion in einer kontinuierlichen Fahrweise durchzuführen.

In einer bevorzugten Ausführungsform der vorliegenden Erfindung ist die Chromatographie, insbesondere Chromatographie an Ionenaustauscherharzen, insbesondere an Kationenaustauscherharzen, in Schritt h) ein einstufiges Verfahren. Bevorzugt ist die Chromatographie, insbesondere Chromatographie an Ionenaustauscherharzen, insbesondere an Kationenaustauscherharzen, in Schritt h) ein mehrstufiges Verfahren, bevorzugt ein zweistufiges Verfahren.

Bevorzugt umfasst die Chromatographie, insbesondere Chromatographie an Ionenaustauscherharzen, insbesondere an Kationenaustauscherharzen, in Schritt h) mehrere Stufen, bevorzugt mindestens zwei Stufen, bevorzugt mindestens drei Stufen, bevorzugt zwei Stufen, bevorzugt drei Stufen.

In einer bevorzugten Ausführungsform der vorliegenden Erfindung kommt es in Schritt h) in einer ersten Stufe der Chromatographie zur Abtrennung von mindestens einer Fraktion, bevorzugt genau einer Fraktion, insbesondere einer HMF-Fraktion oder einer Glucose-Fraktion, bevorzugt von mindestens zwei Fraktionen, bevorzugt genau zwei Fraktionen, bevorzugt genau drei Fraktionen.

In einer weiteren bevorzugten Ausführungsform der vorliegenden Erfindung kommt es in Schritt h) in einer zweiten Stufe der Chromatographie zur Abtrennung von mindestens einer Fraktion, bevorzugt genau einer Fraktion, bevorzugt von mindestens zwei Fraktionen, bevorzugt genau zwei Fraktionen, bevorzugt genau drei Fraktionen, insbesondere einer Glucose-Fraktion, einer Fructose-Fraktion und einer organische Säuren-Fraktion oder einer HMF-Fraktion, einer Fructose-Fraktion und einer organische Säuren-Fraktion.

In einer bevorzugten Ausführungsform der vorliegenden Erfindung handelt es sich bei der ersten Stufe der Chromatographie in Schritt h) um ein Chromatographieverfahren ausgewählt aus der Gruppe bestehend Simulated Moving Bed-Verfahren (SMB), Sequential Simulated Moving Bed-Verfahren (SSMB), Improved Simulated Moving Bed-Verfahren (ISMB) und New MCI-Verfahren (NMCI).

Bevorzugt handelt es sich bei der ersten Stufe der Chromatographie in Schritt h) um ein Improved Simulated Moving Bed-Verfahren (ISMB). Bevorzugt kommt es in Schritt h) in einer ersten Stufe zur Abtrennung von mindestens einer Fraktion, bevorzugt genau einer Fraktion, insbesondere einer HMF-Fraktion oder einer organische Säuren-Fraktion, mittels eines Chromatographieverfahrens ausgewählt aus der Gruppe bestehend Simulated Moving Bed-Verfahren (SMB), Sequential Simulated Moving Bed-Verfahren (SSMB), Improved Simulated Moving Bed-Verfahren (ISMB) und New MCI-Verfahren (NMCI), bevorzugt mittels eines Improved Simulated Moving Bed-Verfahren (ISMB).

In einer bevorzugten Ausführungsform der vorliegenden Erfindung handelt es sich bei der zweiten Stufe der Chromatographie in Schritt h) um ein Chromatographieverfahren ausgewählt aus der Gruppe bestehend Simulated Moving Bed-Verfahren (SMB), Sequential Simulated Moving Bed-Verfahren (SSMB), Improved Simulated Moving Bed-Verfahren (ISMB) und New MCI-Verfahren (NMCI).

Bevorzugt handelt es sich bei der ersten Stufe der Chromatographie in Schritt h) um ein New MCI-Verfahren (NMCI). Bevorzugt kommt es in Schritt h) in einer zweiten Stufe zur Abtrennung von mindestens einer Fraktion, bevorzugt genau einer Fraktion, bevorzugt mindestens zwei Fraktionen, bevorzugt genau zwei Fraktionen, bevorzugt mindestens drei Fraktionen, bevorzugt genau drei Fraktionen, insbesondere einer Glucose-Fraktion, einer Fructose-Fraktion und einer organische Säuren-Fraktion oder einer HMF-Fraktion, einer Fructose-Fraktion und einer organische Säuren-Fraktion, mittels eines Chromatographieverfahrens ausgewählt aus der Gruppe bestehend Simulated Moving Bed-Verfahren (SMB), Sequential Simulated Moving Bed-Verfahren (SSMB), Improved Simulated Moving Bed-Verfahren (ISMB) und New MCI-Verfahren (NMCI), bevorzugt mittels eines New MCI-Verfahrens (NMCI).

Bevorzugt ist insbesondere eine mindestens zweistufige Chromatographie-Auftrennung, bei der in der ersten Stufe die HMF Fraktion abgetrennt wird. Alternativ kann in der ersten Stufe auch die Glucose-Fraktion abgetrennt werden. Bevorzugt ist die erste Stufe der mindestens zweistufigen Chromatographie-Auftrennung ein Moving Bed-Verfahren (ISMB). Bevorzugt ist die zweite Stufe der mindestens zweistufigen Chromatographie-Auftrennung ein New MCI-Verfahrens (NMCI).

Bevorzugt ist insbesondere eine zweistufige Chromatographie-Auftrennung, bei der in der ersten Stufe die HMF-Fraktion abgetrennt wird. Alternativ kann in der ersten Stufe auch die Glucose-Fraktion abgetrennt werden. Bevorzugt ist die erste Stufe der zweistufigen Chromatographie-Auftrennung ein Moving Bed-Verfahren (ISMB). Bevorzugt ist die zweite Stufe der zweistufigen Chromatographie-Auftrennung ein New MCI-Verfahren (NMCI). Bevorzugt werden in der zweiten Stufe der zweistufigen Chromatographie-Auftrennung die organische Säuren-Fraktion, die Fructose-Fraktion und die Glucose-Fraktion voneinander abgetrennt. Alternativ werden in der zweiten Stufe der zweistufigen Chromatographie-Auftrennung die organische Säuren-Fraktion, die Fructose-Fraktion und die HMF-Fraktion voneinander abgetrennt.

In einer bevorzugten Ausführungsform der vorliegenden Erfindung ist die Chromatographie, insbesondere Chromatographie an Ionenaustauscherharzen in Schritt h) eine Chromatographie an Kationenaustauscherharzen.

In einer bevorzugten Ausführungsform der vorliegenden Erfindung wird die Chromatographie, insbesondere Chromatographie an Ionenaustauscherharzen in Schritt h) unter Verwendung eines Kationenaustauscherharzes in H⁺-Form durchgeführt.

In einer bevorzugten Ausführungsform wird die Chromatographie, insbesondere Chromatographie an Ionenaustauscherharzen, in Schritt h) bei einer Temperatur von 40 °C bis 80 °C, bevorzugt 40 °C bis 70 °C, bevorzugt 40 °C bis 60 °C, bevorzugt 50 °C bis 80 °C, bevorzugt 50 °C bis 70 °C, bevorzugt 50 °C bis 60 °C, bevorzugt 60 °C bis 80 °C, bevorzugt 60 °C bis 70 °C, durchgeführt.

Die gegebenenfalls in Schritt i) erhaltene Fructose-Fraktion wird bevorzugt kontinuierlich in den Verfahrensschritt a) rückgeführt. Dabei ist die in Schritt i) gegebenenfalls erhaltene Fructose-Fraktion vorteilhafterweise weitestgehend, bevorzugt vollständig, von gebildeter Lävulinsäure befreit. In einer weiteren bevorzugten Ausführungsform ist die in Schritt i) erhaltene Fructose-Fraktion vorteilhafterweise weitestgehend, bevorzugt vollständig, von gebildeter Lävulin- und Ameisensäure befreit.

In einer besonders bevorzugten Ausführungsform wird die gegebenenfalls in Schritt i) erhaltene Fructose-Fraktion, gegebenenfalls nach Aufkonzentrierung, kontinuierlich bevorzugt vollständig in den Schritt a) rückgeführt. In einer weiteren bevorzugten Ausführungsform wird die in Schritt i) erhaltene Fructose-Fraktion kontinuierlich, gegebenenfalls nach Aufkonzentrierung, zumindest teilweise, insbesondere zu mindestens 70 %, bevorzugt zu mindestens 80 %, bevorzugt zu mindestens 90 %, bevorzugt zu mindestens 95 %, bevorzugt zu mindestens 98 %, bevorzugt zu mindestens 99 %, in Schritt a) rückgeführt (jeweils Gew.-% der rückgeführten Fructose-Fraktion in Bezug auf die in Schritt i) erhaltene Fructose-Fraktion).

Erfindungsgemäß wird unter einer "rückgeführten Fructose-Fraktion" eine nach der gemäß dem erfindungsgemäßen Verfahren durchgeführten Aufreinigung, also Schritt h), gegebenenfalls erhaltene wässrige Fraktion nicht umgesetzter Fructose verstanden, die weitestgehend, bevorzugt vollständig, von während der Fructoseumsetzung gebildeten Nebenprodukten, insbesondere Lävulin- und Ameisensäure und Huminstoffen, befreit ist. Dabei weist die erhaltene wässrige Fraktion nicht umgesetzter Fructose eine derart hohe Reinheit auf, dass sie in einer bevorzugten Ausführungsform direkt, gegebenenfalls nach Aufkonzentrierung, das heißt ohne weitere Aufreinigung, in den Verfahrensschritt a) zurückgeführt wird und nach dem Vermischen mit der Fructose-haltigen Komponente und der katalytisch aktiven Anolytfraktion, das heißt Schritt b), für eine weitere Umsetzung zu HMF in Schritt c) zur Verfügung steht. Besonders bevorzugt sieht daher Schritt a) des erfindungsgemäßen Verfahrens vor eine Fructose-haltige Komponente, eine katalytisch aktive Anolytfraktion und eine rückgeführte Fructose-Fraktion bereitzustellen, die in Schritt b) zum Erhalten einer Reaktionslösung vermischt werden. Da in dieser bevorzugten Ausführungsform bei der Initiierung des erfindungsgemäßen Verfahrens zunächst noch keine rückgeführte Fructose-Fraktion zur Verfügung steht, wird in diesem Fall stattdessen bevorzugt eine entsprechend größere Menge der Fructose-haltigen Komponente verwendet.

In Schritt i) des erfindungsgemäßen Verfahrens, also nach Durchführung der Aufreinigung, wird gegebenenfalls neben der HMF-Fraktion eine Glucose-Fraktion, eine Fructose-Fraktion und eine organische Säuren-Fraktion erhalten, insbesondere isoliert. Vorteilhafterweise weisen die über die verwendeten Aufreinigungsverfahren erhaltenen einzelnen Fraktionen derart hohe Reinheiten auf, dass sie direkt, gegebenenfalls nach Aufkonzentrierung, das heißt ohne weitere Aufreinigung, in unterschiedlichen Folgeprozessen eingesetzt werden können.

Erfindungsgemäß bevorzugt ist die gegebenenfalls erhaltene Fructose-Fraktion weitgehend frei, insbesondere vollständig frei, von gebildeter Lävulinsäure. Erfindungsgemäß bevorzugt ist die erhaltene Fructose-Fraktion weitgehend frei, insbesondere vollständig frei von gebildeten organischen Säuren, insbesondere Lävulin- und Ameisensäure.

Lävulinsäure begünstigt nachteiligerweise die Huminstoffbildung während der HMF-Synthese. So würde ein über die gemäß einer bevorzugten Ausführungsform rückgeführte Fructose-Fraktion bewirkter erhöhter Gehalt an Lävulinsäure in der Reaktionslösung zu einer vermehrten Bildung von Huminstoffen aus HMF und Kohlenhydraten führen und damit die Wirtschaftlichkeit des Prozesses deutlich herabsetzen. Die in dem erfindungsgemäßen Verfahren gegebenenfalls in Schritt i) erhaltene Fructose-Fraktion weist jedoch vorteilhafterweise eine derart hohe Reinheit auf, ist insbesondere frei von gebildeter Lävulinsäure, besonders bevorzugt frei von Lävulin- und Ameisensäure, dass sie in einer bevorzugten Ausführungsform direkt, gegebenenfalls nach Aufkonzentrierung, insbesondere ohne Reinigungsschritte, zur weiteren Umsetzung in das Verfahren, insbesondere Schritt a), zurückgeführt werden kann. Insbesondere kommt es durch die nach dem erfindungsgemäßen Verfahren vorgesehene limitierte Umsetzung von Fructose und der damit verbundenen verringerten Bildung von Neben- und Abbauprodukten, insbesondere Lävulin- und Ameisensäure und Huminstoffen, sowie in bevorzugter Ausführungsform durch die Rückführung einer vom Produktgemisch abgetrennten Fraktion nicht umgesetzter Fructose zur einer hohen HMF-Selektivität und einer hohen HMF-Ausbeute.

Überraschenderweise ergibt sich bei der erfindungsgemäßen Verfahrensführung ein inverser Zusammenhang zwischen der Fructosereinheit und der HMF-Selektivität, das heißt, mit abnehmendem Fructoseanteil in der Reaktionslösung steigt die Selektivität für HMF.

In einer besonders bevorzugten Ausführungsform besteht das erfindungsgemäße Verfahren aus den Verfahrensschritten a), b), c) und d), insbesondere werden zwischen diesen Verfahrensschritten keine weiteren Verfahrensschritte durchgeführt.

In besonders bevorzugter Ausführungsform der vorliegenden Erfindung umfasst das erfindungsgemäße Verfahren die Verfahrensschritte a), b), c) und d), wobei zwischen den Verfahrensschritten a), b), c) und d) keine weiteren Verfahrensschritte durchgeführt werden, optional allerdings nach Durchführung von Verfahrensschritt d) weitere Verfahrensschritte durchgeführt werden.

Erfindungsgemäß umfasst das vorliegende Verfahren die Schritte a) bis d), bevorzugt a) bis e), bevorzugt a) bis f), bevorzugt a) bis g), bevorzugt a) bis h), insbesondere a) bis i). Erfindungsgemäß besonders bevorzugt umfasst das vorliegende Verfahren die Schritte a), b), c), d), e), f), g), h) und i). Es kann jedoch auch vorgesehen sein, dass das vorliegende Verfahren die Schritte a), b), c), d), e), h) und i) oder a), b), c), d), e), f), h) und i) oder a), b), c), d), e), g) h) und i) umfasst. In besonders bevorzugter Ausführungsform besteht das vorliegende Verfahren aus den Verfahrensschritten a) bis d), bevorzugt a) bis e), bevorzugt a) bis f), bevorzugt a) bis g), bevorzugt a) bis h), insbesondere a) bis i). In besonders bevorzugter Ausführungsform besteht das vorliegende Verfahren aus den Verfahrensschritten a), b), c), d), e), h) und i) oder a), b), c), d), e), f), h) und i) oder a), b), c), d), e), g) h) und i). In bevorzugter Ausführungsform wird das Verfahren in der Reihenfolge der Verfahrensschritte a), b), c), d), e), f), g), h) und i) durchgeführt. Es kann jedoch auch vorgesehen sein, dass das vorliegende Verfahren in der Reihenfolge der Verfahrensschritte a), b), c), d), e), h) und i) oder a), b), c), d), e), f), h) und i) oder a), b), c), d), e), g) h) und i) durchgeführt wird.

Erfindungsgemäß erfolgt im Verfahren zur Herstellung von 5-Hydroxymethylfurfural gemäß der Schritte a) bis i), die Umsetzung der in der Reaktionsmischung vorhandenen Fructose zu HMF in einem kontinuierlichen Reaktorsystem und die anschließende Aufreinigung der erhaltenen Produktmischung zur Auftrennung von mindestens vier Fraktionen kontinuierlich, das heißt unter konstanter Zuführung von Edukten und Entnahme von Produkten.

Bevorzugt wird unter einem kontinuierlichen erfindungsgemäßen Prozess ein Prozess verstanden, bei dem nicht nur das Reaktorsystem kontinuierlich ist, sondern auch die Aufreinigung der Produktmischung.

Die vorliegende Erfindung ermöglicht die Bereitstellung von Verfahren zur Herstellung von HMF und/oder Ameisensäure und/oder Lävulinsäure, insbesondere zur simultanen Herstellung aus einem Ausgangsmaterial, nämlich einer Fructose-haltigen Komponente und gegebenenfalls einer rückgeführten Fructose-Fraktion.

Das erfindungsgemäße Verfahren zur Herstellung von HMF ist in bevorzugter Ausführungsform daher auch ein Verfahren zur Herstellung von HMF und Ameisensäure und Lävulinsäure, welches die Schritte a) bis i), umfasst und der gezielten Herstellung dreier interessierender Produkte dient.

Das erfindungsgemäße Verfahren zur Herstellung von HMF ist in bevorzugter Ausführungsform daher auch ein Verfahren zur Herstellung von HMF und Ameisensäure, welches die Schritte a) bis i), umfasst und das zur Herstellung von zwei interessierenden Wertstoffen dient.

Das erfindungsgemäße Verfahren zur Herstellung von HMF ist in bevorzugter Ausführungsform daher auch ein Verfahren zur Herstellung von HMF und Lävulinsäure, welches die Schritte a) bis i), umfasst und das zur Herstellung von zwei interessierenden Wertstoffen dient.

Erfindungsgemäß umfasst die in Schritt i) erhaltene Glucose-Fraktion mindestens 20 Gew.-% der in der Produktmischung enthaltenen Glucose (jeweils TS bezogen auf Produktmischung).

In einer weiteren bevorzugten Ausführungsform der vorliegenden Erfindung weist die gegebenenfalls in Schritt i) erhaltene Glucose-Fraktion eine hinreichend hohe Reinheit auf, ist insbesondere frei von Fermentationsinhibitoren, dass sie direkt, gegebenenfalls nach Aufkonzentrierung, sowohl als Feed (Einspeisungsmaterial) in fermentativen Prozessen, insbesondere zur Ethanolherstellung, insbesondere Glucose-Fermentation zu Ethanol, als auch als Edukt in chemischen Prozessen, insbesondere der Oxidation von Glucose zu Gluconsäure, eingesetzt werden kann.

In einer weiteren bevorzugten Ausführungsform wird die gegebenenfalls in Schritt i) erhaltene, Glucose-Fraktion für die Ethanolherstellung, insbesondere Glucose-Fermentation zu Ethanol, insbesondere für die Bio-Ethanol-Gewinnung, und/oder zur Gluconsäure-Gewinnung eingesetzt.

Die vorliegende Erfindung stellt daher auch ein Verfahren zur Herstellung eines Feeds für fermentative Prozesse, insbesondere zur Ethanolherstellung, insbesondere Glucose-Fermentation zu Ethanol, oder zur Herstellung eines Ausgangsmaterials, das heißt Eduktes, in chemischen Prozessen, insbesondere zur Herstellung von Gluconsäure, bereit, im Rahmen dessen ein Verfahren der vorliegenden Erfindung mit den Verfahrensschritten a) bis i) unter Erhalten einer Glucose-Fraktion, die als Feed oder Edukt eingesetzt werden kann, durchgeführt wird.

In einer besonders bevorzugten Ausführungsform wird ein Verfahren für die Ethanolherstellung, insbesondere Glucose-Fermentation zu Ethanol bereitgestellt, im Rahmen dessen das erfindungsgemäße Verfahren, insbesondere die Verfahrensschritte a) bis i), insbesondere zum Erhalten einer Glucose-Fraktion durchgeführt werden, wobei die erhaltene Glucose-Fraktion für die Ethanolherstellung, insbesondere Glucose-Fermentation zu Ethanol, insbesondere für die Bio-Ethanol-Gewinnung, eingesetzt wird.

In einer weiteren bevorzugten Ausführungsform wird die gegebenenfalls in Schritt i) erhaltene Glucose-Fraktion zur Gluconsäure-Gewinnung eingesetzt, gegebenenfalls nach Aufkonzentrierung.

In einer besonders bevorzugten Ausführungsform wird ein Verfahren zur Herstellung von Gluconsäure bereitgestellt, dass das erfindungsgemäße Verfahren umfasst, insbesondere die Verfahrensschritte a) bis i), insbesondere zum Erhalten einer Glucose-Fraktion, die zur Gewinnung von Glucose und zur anschließenden Oxidation der Glucose zu Gluconsäure eingesetzt wird.

In einer bevorzugten Ausführungsform der vorliegenden Erfindung wird die gegebenenfalls in Schritt i) erhaltene organische Säuren-Fraktion zur Isolierung von Lävulin- und Ameisensäure eingesetzt. In einer weiteren bevorzugten Ausführungsform wird die in Schritt i) erhaltene organische Säuren-Fraktion zur Isolierung von Lävulinsäure eingesetzt. In einer weiteren bevorzugten Ausführungsform wird die in Schritt i) erhaltene organische Säuren-Fraktion zur Isolierung von Ameisensäure eingesetzt.

Die vorliegende Erfindung betrifft daher auch ein Verfahren zur Herstellung von Lävulinsäure, Ameisensäure oder Lävulinsäure und Ameisensäure, wobei ein Verfahren, umfassend die Schritte a) bis i), der vorliegenden Erfindung durchgeführt wird und in einem Schritt i) Lävulinsäure, Ameisensäure oder Lävulinsäure und Ameisensäure erhalten wird.

In einer weiteren bevorzugten Ausführungsform der vorliegenden Erfindung wird die in Schritt i) erhaltene HMF-Fraktion direkt, gegebenenfalls nach Aufkonzentrierung, das heißt ohne die Notwendigkeit aufwendiger weiterer Aufreinigung, in einem zusätzlichen Schritt zu 2,5-Furandicarbonsäure (FDCA) oxidiert.

Die vorliegende Erfindung betrifft daher auch ein Verfahren zur Herstellung von FDCA, umfassend die Schritte a) bis i) der vorliegenden Erfindung, wobei die in Schritt i) erhaltene HMF-Fraktion, vorzugsweise direkt, gegebenenfalls nach Aufkonzentrierung, und ohne die Notwendigkeit aufwendiger weiterer Aufreinigung, zu FDCA oxidiert wird.

Erfindungsgemäß enthält die gegebenenfalls erhaltene Glucose-Fraktion 0,8 Gew.-% bis 100 Gew.-% Glucose, 0 Gew.-% bis 99,2 Gew.-% Fructose, höchstens 2 Gew.-%, bevorzugt höchstens 1 Gew.-%, bevorzugt höchstens 0,5 Gew.-%, bevorzugt höchstens 0,1 Gew.-%, Lävulin- und Ameisensäure und höchstens 10 Gew.-%, bevorzugt höchstens 5 Gew.-%, bevorzugt höchstens 2 Gew.-%, mehr bevorzugt höchstens 1 Gew.-%, bevorzugt höchstens 0,5 Gew.-%, bevorzugt höchstens 0,1 Gew.-%, HMF (jeweils TS, bezogen auf Summe der analysierten Komponenten (Glucose, Fructose, Lävulinsäure, Ameisensäure, HMF, Difructoseanhydride (DFA))). Erfindungsgemäß bevorzugt enthält die Glucose-Fraktion höchstens 10 Gew.-%, mehr bevorzugt höchstens 5 Gew.-% HMF.

Die gegebenenfalls in Schritt i) erhaltene Fructose-Fraktion enthält erfindungsgemäß mindestens 70 Gew.-%, bevorzugt mindestens 80 Gew.-%, der in der Produktmischung enthaltenen Fructose (jeweils TS bezogen auf Produktmischung).

Erfindungsgemäß enthält die gegebenenfalls erhaltene Fructose-Fraktion 0 Gew.-% bis 60 Gew.% Glucose, 40 Gew.-% bis 100 Gew.-% Fructose, höchstens 2 Gew.-%, bevorzugt höchstens 1 Gew.-%, bevorzugt höchstens 0,5 Gew.-%, bevorzugt höchstens 0,1 Gew.-% Lävulinsäure, höchstens 2 Gew.-%, bevorzugt höchstens 1,5 Gew.-%, bevorzugt höchstens 1 Gew.-%, bevorzugt höchstens 0,5 Gew.-%, bevorzugt höchstens 0,25 Gew.-%, bevorzugt höchstens 0,1 Gew.-%, Ameisensäure und höchstens 2 Gew.-%, bevorzugt höchstens 1,5 Gew.-%, bevorzugt höchstens 1 Gew.-%, bevorzugt höchstens 0,8 Gew.-%, bevorzugt höchstens 0,6 Gew.-%, bevorzugt höchstens 0,4 Gew.-%, bevorzugt höchstens 0,2 Gew.-%, bevorzugt höchstens 0,1 Gew.-%, HMF (jeweils TS, bezogen auf die Summe der analysierten Komponenten (Glucose, Fructose, Lävulinsäure, Ameisensäure, HMF, Difructoseanhydride (DFA)). Erfindungsgemäß bevorzugt enthält die Fructose-Fraktion höchstens 2 Gew.-%, HMF. Erfindungsgemäß bevorzugt enthält die Fructose-Fraktion höchstens 2 Gew.-%, Lävulinsäure. In besonders bevorzugter Ausführungsform ist das Verhältnis von Fructose zu Glucose in der Fructose-Fraktion nicht kleiner als in der in Schritt a) bereitgestellten Fructose-haltigen Komponente.

Erfindungsgemäß enthält die gegebenenfalls in Schritt i) erhaltene organische Säuren-Fraktion mindestens 60 Gew.-%, bevorzugt mindestens 65 Gew.-%, bevorzugt mindestens 70 Gew.-%, bevorzugt mindestens 80 Gew.-%, bevorzugt mindestens 90 Gew.-%, bevorzugt mindestens 95 Gew.-%, bevorzugt mindestens 98 Gew.-%, bevorzugt mindestens 99 Gew.-%, bevorzugt mindestens 99,5 Gew.-%, bevorzugt mindestens 99,8 Gew.-%, bevorzugt 100 Gew.-% der in der Produktmischung enthaltenen Lävulin- und Ameisensäure (jeweils TS, bezogen auf Produktmischung).

Erfindungsgemäß enthält die gegebenenfalls erhaltene organische Säuren-Fraktion 50 Gew.-% bis 100 Gew.-%, bevorzugt 60 Gew.-% bis 100 Gew.-%, bevorzugt, mehr bevorzugt 65 Gew.-% bis 100 Gew.-%, bevorzugt 70 Gew.-% bis 100 Gew.-%, bevorzugt 80 Gew.-% bis 100 Gew.-%, bevorzugt 90 Gew.-% bis 100 Gew.-%, bevorzugt 95 Gew.-% bis 100 Gew.-%, bevorzugt 98 Gew.-% bis 100 Gew.-%, bevorzugt 99 Gew.-% bis 100 Gew.-%, bevorzugt 99,5 Gew.-% bis 100 Gew.-%, bevorzugt 99,7 Gew.-% bis 100 Gew.-% Lävulin- und Ameisensäure (jeweils TS, bezogen auf Summe der analysierten Komponenten (Glucose, Fructose, Lävulinsäure, Ameisensäure, HMF, Difructoseanhydride (DFA)). Erfindungsgemäß bevorzugt enthält die organische Säuren-Fraktion mindestens 50 Gew.-% Lävulinsäure, mehr bevorzugt mindestens 60 Gew.-% Lävulinsäure, mehr bevorzugt mindestens 70 Gew.-% Lävulinsäure.

Die in Schritt i) erhaltene HMF-Fraktion enthält erfindungsgemäß mindestens 70 Gew.-%, bevorzugt mindestens 80 Gew.-%, mehr bevorzugt mindestens 90 Gew.-%, bevorzugt mindestens 98 Gew.-%, bevorzugt mindestens 99 Gew.-%, bevorzugt mindestens 99,5 Gew.-%, bevorzugt mindestens 99,8 Gew.-%, bevorzugt 100 Gew.-% des in der Produktmischung enthaltenen HMF (jeweils TS, bezogen auf Produktmischung).

Erfindungsgemäß enthält die HMF-Fraktion 80 Gew.-% bis 100 Gew.-%, bevorzugt 85 Gew.-% bis 100 Gew.-%, bevorzugt 90 Gew.-% bis 100 Gew.-%, bevorzugt 95 Gew.-% bis 100 Gew.-%, bevorzugt 98 Gew.-% bis 100 Gew.-%, bevorzugt 99 Gew.-% bis 100 Gew.-%, bevorzugt 99,5 Gew.-% bis 100 Gew.-%, bevorzugt 99,7 Gew.-% bis 100 Gew.-% HMF und höchstens 16 Gew.-%, bevorzugt höchstens 14 Gew.-%, bevorzugt höchstens 12 Gew.-%, bevorzugt höchstens 10 Gew.-%, bevorzugt höchstens 8 Gew.-%, bevorzugt höchstens 6 Gew.-%, bevorzugt höchstens 4 Gew.-%, bevorzugt höchstens 2 Gew.-%, bevorzugt höchstens 1 Gew.-%, Lävulin- und Ameisensäure, höchstens 2 Gew.-%, bevorzugt höchstens 1 Gew.-%, bevorzugt höchstens 0,8 Gew.-%, bevorzugt höchstens 0,6 Gew.-%, bevorzugt höchstens 0,4 Gew.-%, bevorzugt höchstens 0,2 Gew.-%, bevorzugt höchstens 0,1 Gew.-%, Glucose und höchstens 2 Gew.-%, bevorzugt höchstens 1 Gew.-%, bevorzugt höchstens 0,8 Gew.-%, bevorzugt höchstens 0,6 Gew.-%, bevorzugt höchstens 0,4 Gew.-%, bevorzugt höchstens 0,2 Gew.-%, bevorzugt höchstens 0,1 Gew.-% Fructose (jeweils TS, bezogen auf Summe der analysierten Komponenten (Glucose, Fructose, Lävulinsäure, Ameisensäure, HMF, Difructoseanhydride (DFA)).

In einer bevorzugten Ausführungsform werden in dem erfindungsgemäßen Verfahren, insbesondere während der Schritte a) bis g), gegebenenfalls a) bis i), keine organischen Lösungsmittel, insbesondere keine ionischen Flüssigkeiten eingesetzt.

In einer bevorzugten Ausführungsform wird das erfindungsgemäße Verfahren, insbesondere während der Schritte a) bis i), nicht unter sauerstoffreduzierten Bedingungen durchgeführt.

Unter dem Begriff "und/oder" wird in Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind. Dies bedeutet für den Ausdruck "A, B und/oder C", dass folgender Offenbarungsgehalt darunter zu verstehen ist: A oder B oder C oder (A und B) oder (A und C) oder (B und C) oder (A und B und C).

Im Zusammenhang mit der vorliegenden Erfindung wird unter dem Begriff "umfassend" verstanden, dass zusätzlich zu den von dem Begriff explizit erfassten Elementen noch weitere, nicht explizit genannte Elemente hinzutreten können. Im Zusammenhang mit der vorliegenden Erfindung wird unter diesen Begriffen auch verstanden, dass allein die explizit genannten Elemente erfasst werden und keine weiteren Elemente vorliegen. In dieser besonderen Ausführungsform ist die Bedeutung des Begriffes "umfassend" gleichbedeutend mit dem Begriff "bestehend aus". Darüber hinaus erfasst der Begriff "umfassend" auch Gesamtheiten, die neben den explizit genannten Elementen auch weitere nicht genannte Elemente enthalten, die jedoch von funktionell und qualitativ untergeordneter Natur sind. In dieser Ausführungsform ist der Begriff "umfassend" gleichbedeutend mit dem Begriff "im Wesentlichen bestehend aus".

Weitere bevorzugte Ausführungsformen ergeben sich aus den Unteransprüchen.

Die Erfindung wird anhand der folgenden Ausführungsbeispiele und der dazugehörigen Figuren näher erläutert.

Die Figuren zeigen:

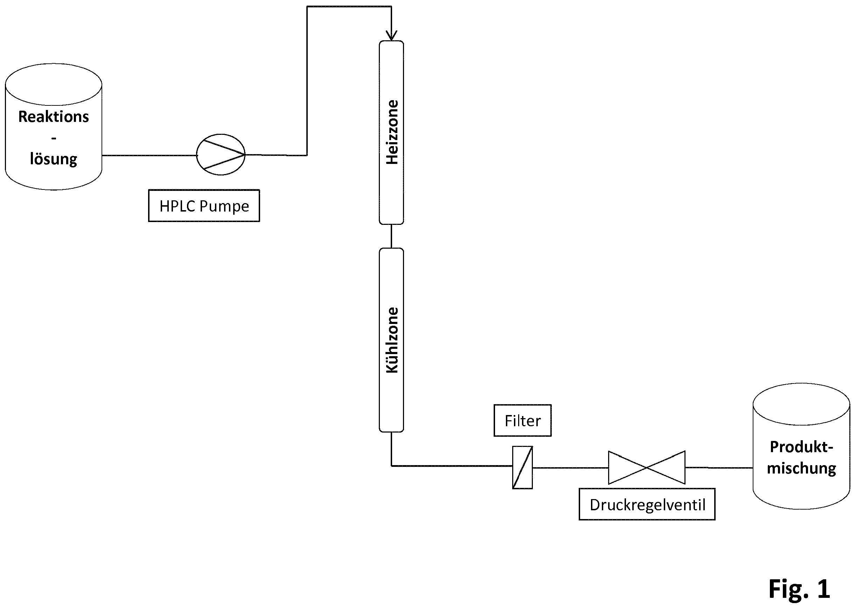

Figur 1 zeigt eine schematische Darstellung des erfindungsgemäß verwendeten Reaktorsystems.

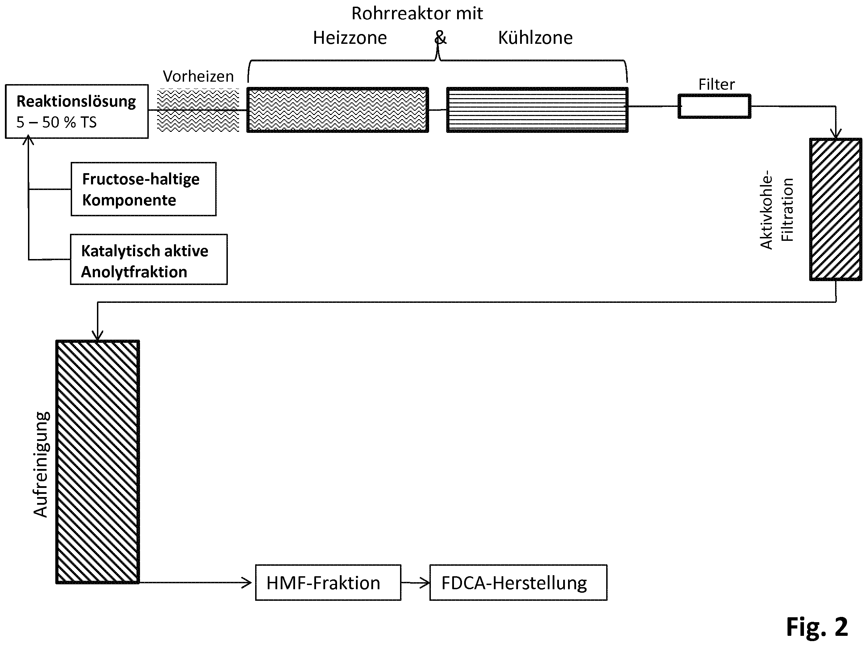

Figur 2 zeigt eine schematische Darstellung des erfindungsgemäßen Verfahrens, wobei die in Schritt a) bereitgestellten Komponenten zunächst in Schritt b) vermischt und die erhaltene Reaktionslösung anschließend erhitzt wird und nach dem Aufreinigungsschritt h) eine HMF-Fraktion erhalten wird (Schritt i)).

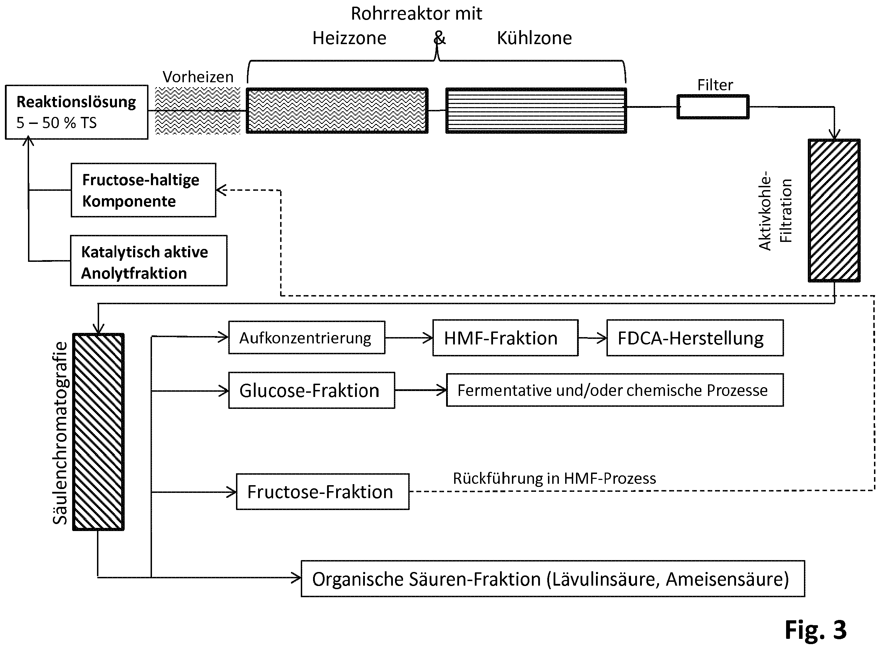

Figur 3 zeigt eine schematische Darstellung des erfindungsgemäßen Verfahrens analog zu Figur 2, wobei in Schritt h) eine säulenchromatographische Auftrennung durchgeführt wird und eine HMF-Fraktion, eine Glucose-Fraktion, eine Fructose-Fraktion und eine organische Säuren-Fraktion erhalten wird (Schritt i)).

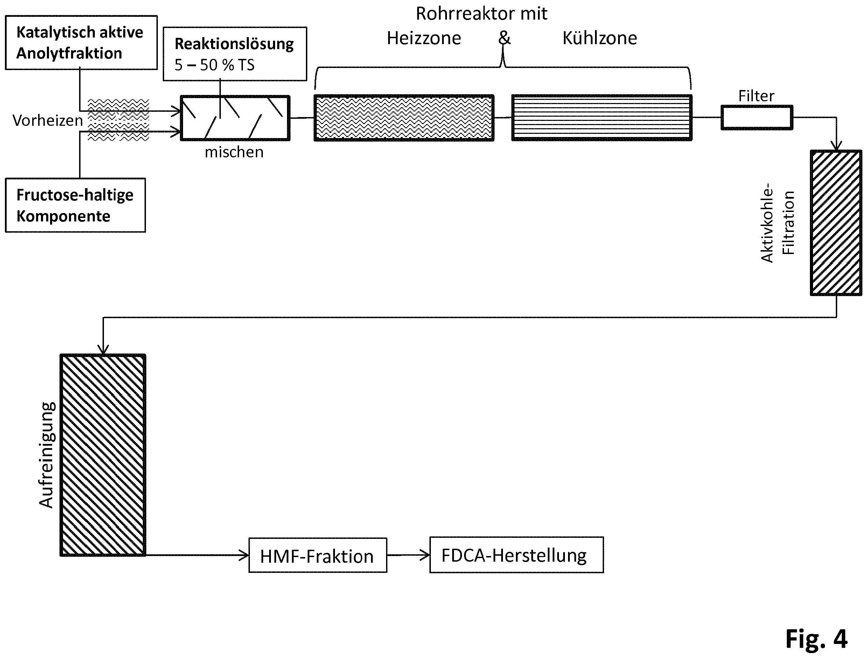

Figur 4 zeigt eine schematische Darstellung des erfindungsgemäßen Verfahrens, wobei die in Schritt a) bereitgestellten Komponenten getrennt voneinander erhitzt und erst anschließend in Schritt b) zum Erhalten einer Reaktionslösung vermischt werden und nach dem Aufreinigungsschritt h) eine HMF-Fraktion erhalten wird (Schritt i)).

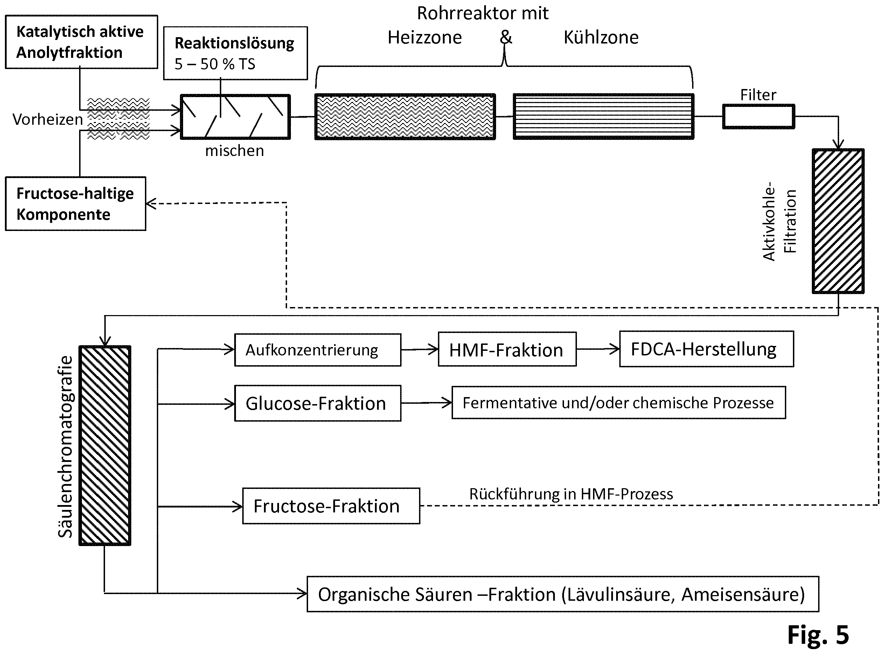

Figur 5 zeigt eine schematische Darstellung des erfindungsgemäßen Verfahrens analog zu Figur 4, wobei in Schritt h) eine säulenchromatographische Auftrennung durchgeführt wird und eine HMF-Fraktion, eine Glucose-Fraktion, eine Fructose-Fraktion und eine organische Säuren-Fraktion erhalten wird (Schritt i)).

### Beispiele

Im erfindungsgemäßen Verfahren werden als Ausgangsmaterialien eine Fructose-haltige Komponente, die ein variables Verhältnis von Fructose zu Glucose aufweist und eine katalytisch aktive Anolytfraktion eingesetzt. Die katalytisch aktive Anolytfraktion wird in einem Wasserionisierer (Titanion SE Ultra) aus mit dem entsprechenden Elektrolyten versetzten VE-Wasser hergestellt. Die Fructose-haltige Komponente wird mit der katalytisch aktiven Anolytfraktion vermischt, sodass eine Reaktionslösung mit einem Trockensubstanzgehalt von 20 bis 40 Gew.-% (TS Kohlenhydrat bezogen auf Gesamtgewicht Reaktionslösung) erhalten wird. Die so erhaltene Reaktionslösung wird mit Hilfe einer HPLC-Pumpe in die beheizte "Heizzone" des Rohrreaktors (Außendurchmesser 8 mm, Innendurchmesser 6 mm, Länge 630 mm) gepumpt und dort umgesetzt. Der Rohrreaktor ist als Doppelrohrwärmetauscher im Gegenstrom ausgestaltet, wobei die Temperierung mittels eines Thermoöls im Außenmantel erfolgt. Das Thermoöl wird mittels eines Thermostaten temperiert. Nach der "Heizzone" erfolgt direkt der Übergang in die "Kühlzone". Diese ist ebenfalls als Doppelrohrwärmetauscher im Gegenstrom ausgeführt (Außendurchmesser des produktführenden Rohres 8 mm, Innendurchmesser 6 mm, Länge 125 mm). Innerhalb der "Kühlzone" wird die Reaktionslösung auf Raumtemperatur abgekühlt und die Umsetzung gestoppt. Im Anschluss wird die Produktmischung über einen Metallsinterfilter (Porenweite 7 µm) filtriert und von eventuell entstandenen unlöslichen Huminstoffen befreit. Der Druck im Reaktorsystem wird mit Hilfe eines Druckhalteventils so eingestellt, dass ein Sieden der Reaktionslösung und damit das Auftreten von Dampfblasen vermieden wird (ca. 1 MPa bei 180 °C).

Die nachfolgenden Beispiele zeigen die Durchführung des erfindungsgemäßen Verfahrens mit verschiedenen Fructose-haltige Komponenten, unterschiedlich hergestellten katalytisch aktiven Anolytfraktionen, verschiedenen TS-Gehalten und bei unterschiedlichen Temperaturen.

Bei allen Experimenten wurden während des Versuchs Proben entnommen und mittels HPLC analysiert (BIORAD Aminex 87-H, 5 mmol/I Schwefelsäure, 50 °C). Aus den Analysenergebnissen wurde anschließend Fructoseumsatz, HMF-Selektivität und die Bilanz berechnet (Bilanz = (Summe nicht-umgesetzte Zucker, HMF und Ameisensäure (in mol) ^{∗} 100 / eingesetzter Zucker (in mol)). Lävulinsäure wird in der Bilanz nicht berücksichtigt, da je ein Molekül Ameisensäure und Lävulinsäure aus einem Molekül HMF entstehen.

### Beispiel 1: HMF-Synthese mit katalytisch aktiver Anolytfraktion auf Basis von 0,18 % NaCl bei 170 °C (MMR 112)

Als Fructose-haltige Komponente wurde ein Fructosesirup mit 95 % Fructosereinheit und einem TS-Gehalt von 70 % eingesetzt. Die katalytisch aktive Anolytfraktion wurde aus einer Elektrolytlösung hergestellt, die einen Gehalt von 0,18 Gew.-% Natriumchlorid enthielt und hatte einen pH-Wert von 2,3. Der Fructosesirup wurde mit der katalytisch aktiven Anolytfraktion auf einen TS-Gehalt von 20 % TS Kohlenhydrat verdünnt. Diese Reaktionslösung wurde dann mit einer Verweilzeit von 5,6 min in der Heizzone bei einer Temperatur von 170 °C (Temperatur des Thermoöls) umgesetzt.
Fructose-Umsatz: 31,6 %
HMF-Selektivität: 81,2 %
Ameisensäure-Selektivität: 4,5 %
Lävulinsäure-Selektivität: 2,3 %
Bilanz: 96 %

### Beispiel 2: HMF-Synthese mit katalytisch aktiver Anolytfraktion auf Basis von 0,625 % NaCl bei 170 °C (MMR 114)

Als Fructose-haltige Komponente wurde ein Fructosesirup mit 95 % Fructosereinheit und einem TS-Gehalt von 70 % eingesetzt. Die katalytisch aktive Anolytfraktion wurde aus einer Elektrolytlösung hergestellt, die einen Gehalt von 0,625 Gew.-% Natriumchlorid enthielt und hatte einen pH-Wert von 2,3. Der Fructosesirup wurde mit der katalytisch aktive Anolytfraktion auf einen TS-Gehalt von 20 % TS Kohlenhydrat verdünnt. Diese Reaktionslösung wurde dann mit einer Verweilzeit von 5,6 min in der Heizzone bei einer Temperatur von 170 °C (Temperatur des Thermoöls) umgesetzt.
Fructose-Umsatz: 35,0 %
HMF-Selektivität: 80,6 %
Ameisensäure-Selektivität: 5,2 %
Lävulinsäure-Selektivität: 2,3 %
Bilanz: 95,6 %

### Beispiel 3: HMF-Synthese mit katalytisch aktiver Anolytfraktion auf Basis von 1 % NaNO₃ bei 165 °C (MMR 117)

Als Fructose-haltige Komponente wurde ein Fructosesirup mit 95 % Fructosereinheit und einem TS-Gehalt von 70 % eingesetzt. Die katalytisch aktive Anolytfraktion wurde aus einer Elektrolytlösung hergestellt, die einen Gehalt von 1,0 Gew.-% Natriumnitrat enthielt und hatte einen pH-Wert von 2,2. Der Fructosesirup wurde mit der katalytisch aktiven Anolytfraktion auf einen TS-Gehalt von 20 % TS Kohlenhydrat verdünnt. Diese Reaktionslösung wurde dann mit einer Verweilzeit von 5,6 min in der Heizzone bei einer Temperatur von 165 °C (Temperatur des Thermoöls) umgesetzt.
Fructose-Umsatz: 19,97 %
HMF-Selektivität: 81,8 %
Ameisensäure-Selektivität: 2,9 %
Lävulinsäure-Selektivität: 1,6 %
Bilanz: 97,23 %

### Beispiel 4: HMF-Synthese mit katalytisch aktiver Anolytfraktion auf Basis von 0,25 % NaNO₃ bei 165 °C (MMR 117/2)

Als Fructose-haltige Komponente wurde ein Fructosesirup mit 95 % Fructosereinheit und einem TS-Gehalt von 70 % eingesetzt. Die katalytisch aktive Anolytfraktion wurde aus einer Elektrolytlösung hergestellt, die einen Gehalt von 0,25 Gew.-% Natriumnitrat enthielt und hatte einen pH-Wert von 2,2. Der Fructosesirup wurde mit der katalytisch aktive Anolytfraktion auf einen TS-Gehalt von 20 % TS Kohlenhydrat verdünnt. Diese Reaktionslösung wurde dann mit einer Verweilzeit von 5,6 min in der Heizzone bei einer Temperatur von 165 °C (Temperatur des Thermoöls) umgesetzt.
Fructose-Umsatz: 16,5 %
HMF-Selektivität: 86,5 %
Ameisensäure-Selektivität: 3,1%
Lävulinsäure-Selektivität: 1,5 %
Bilanz: 98,6 %

### Beispiel 5: HMF-Synthese mit katalytisch aktiver Anolytfraktion auf Basis von 0,18 % NaCl bei 165 °C mit 30 % TS (MMR 137)

Als Fructose-haltige Komponente wurde ein Fructosesirup mit 85 % Fructosereinheit und einem TS-Gehalt von 75 % eingesetzt. Die katalytisch aktive Anolytfraktion wurde aus einer Elektrolytlösung hergestellt, die einen Gehalt von 0,18 Gew.-% Natriumchlorid enthielt und hatte einen pH-Wert von 2,3. Der Fructosesirup wurde mit der katalytisch aktiven Anolytfraktion auf einen TS-Gehalt von 30 % TS Kohlenhydrat verdünnt. Diese Reaktionslösung wurde dann mit einer Verweilzeit von 5,6 min in der Heizzone bei einer Temperatur von 165 °C (Temperatur des Thermoöls) umgesetzt.
Fructose-Umsatz: 17,6 %
HMF-Selektivität: 88,2 %
Ameisensäure-Selektivität: 4,7 %
Lävulinsäure-Selektivität: 2,1 %
Bilanz: 97,9 %

### Beispiel 6: HMF-Synthese mit katalytisch aktiver Anolytfraktion auf Basis von 0,18 % NaCl bei 165 °C mit 40 % TS (MMR 136)

Als Fructose-haltige Komponente wurde ein Fructosesirup mit 85 % Fructosereinheit und einem TS-Gehalt von 75 % eingesetzt. Die katalytisch aktive Anolytfraktion wurde aus einer Elektrolytlösung hergestellt, die einen Gehalt von 0,18 Gew.-% Natriumchlorid enthielt und hatte einen pH-Wert von 2,3. Der Fructosesirup wurde mit der katalytisch aktiven Anolytfraktion auf einen TS-Gehalt von 40 %TS Kohlenhydrat verdünnt. Diese Reaktionslösung wurde dann mit einer Verweilzeit von 5,6 min in der Heizzone bei einer Temperatur von 165 °C (Temperatur des Thermoöls) umgesetzt.
Fructose-Umsatz: 15,5 %
HMF-Selektivität: 89,7 %
Ameisensäure-Selektivität: 5,5 %
Lävulinsäure-Selektivität: 1,6 %
Bilanz: 98,2 %

### Beispiel 7: Einfluss der Fructosereinheit auf die HMF-Selektivität in der HMF-Synthese mit katalytisch aktiver Anolytfraktion

Als Fructose-haltige Komponenten wurden Fructoselösungen mit unterschiedlichen Fructosereinheiten (62 %, 70 %, 80 %, 85 %, 90 % und 100 %) und mit einem TS-Gehalt von 30 % in katalytisch aktiver Anolytfraktion hergestellt. Die katalytisch aktive Anolytfraktion wurde aus einer Elektrolytlösung hergestellt, die einen Gehalt von 0,18 Gew.-% Natriumchlorid enthielt und hatte einen pH-Wert von 2,3. Diese Reaktionslösungen wurden dann jeweils mit einer Verweilzeit von 5,6 min in der Heizzone bei einer Temperatur von 165 °C (Temperatur des Thermoöls) umgesetzt.

| Fructosereinheit [%] | FructoseUmsatz [%] | HMF-Selektivität [%] | Ameisensäure-Selektivität [%] | Lävulinsäure-Selektivität [%] | Bilanz [%] |
|---|---|---|---|---|---|
| 62 | 17.8 | 89.3 | 5.8 | 1.5 | 96.2 |
| 70 | 18,3 | 89,0 | 3,9 | 1,4 | 97.5 |
| 80 | 18.2 | 88,5 | 3.0 | 1.6 | 98.4 |
| 85 | 17,9 | 88,3 | 4.7 | 2.0 | 97,9 |
| 90 | 17.8 | 83.8 | 2.4 | 1,1 | 97,9 |
| 95 | 18,0 | 81,2 | 2,8 | 1,4 | 97,9 |
| 100 | 18,1 | 80,1 | 3,1 | 1,7 | 97,6 |

Mit zunehmender Reinheit der Fructose wird die Selektivität zu HMF bei gleichem Umsatz schlechter.

### Beispiel 8: Einfluss des Kations bei der Herstellung der katalytisch aktiven Anolytfraktion auf die HMF-Selektivität in der HMF-Synthese

Als Fructose-haltige Komponente wurde ein Fructosesirup mit einer Fructosereinheit von 85 % und einem TS-Gehalt von 75 % eingesetzt. Dieser Sirup wurde jeweils mit einer katalytisch aktiven Anolytfraktion auf Basis verschiedener Chlorid-Salze (Lithium-, Natrium-, KaliumChlorid, jeweils 0,18 Gew.-%) auf einen Trockensubstanzgehalt von 30 % Kohlenhydrat verdünnt.

Diese Reaktionslösungen wurden dann jeweils mit einer Verweilzeit von 5,6 min in der Heizzone bei einer Temperatur von 165 °C (Temperatur des Thermoöls) umgesetzt.

| Elektrolyt | pH-Wert katalytisch aktive Anolytfraktion [-] | Chloridgehalt katalytisch aktive Anolytfraktion [mg/l] | Fructose - Umsatz [%] | HMF-Selektivität [%] | Ameisensäure-Selektivität [%] | Lävulinsäure-Selektivität [%] | Bilanz [%] |
|---|---|---|---|---|---|---|---|
| LiCl | 2,2 | 1101 | 17,9 | 87,9 | 4,7 | 2,0 | 97,7 |
| NaCl | 2,3 | 793 | 17,6 | 88,2 | 4,7 | 2,1 | 97,9 |
| KCl | 2,3 | 645 | 17,2 | 86,8 | 2,9 | 1,1 | 97,9 |

### Beispiel 9: Vergleichsexperiment mit 0,75 % H₂SO₄ (ohne katalytisch aktive Anolytfraktion, Stand der Technik) bei 135 °C und 30 % TS (MMR 149)

Als Fructose-haltige Komponente wurde ein Fructosesirup mit einer Fructosereinheit von 85 % und einem TS-Gehalt von 75 % eingesetzt. Dieser Sirup wurde mit VE-Wasser auf einen Trockensubstanzgehalt von 30 % Kohlenhydrat eingestellt und mit 0,75 % Schwefelsäure versetzt.

Diese Reaktionslösung wurde dann jeweils mit einer Verweilzeit von 5,6 min in der Heizzone bei einer Temperatur von 135 °C (Temperatur des Thermoöls) umgesetzt.
Fructose-Umsatz: 20,0 %
HMF-Selektivität: 73,2 %
Ameisensäure-Selektivität: 9,15 %
Bilanz: 96,1 %

### Beispiel 10: Einfluss der Chloridkonzentration auf Umsatz und HMF-Selektivität

Als Fructose-haltige Komponente wurde ein Fructosesirup mit einer Fructosereinheit von 95 % und einem TS-Gehalt von 75 % eingesetzt. Dieser Sirup wurde jeweils mit katalytisch aktiven Anolytfraktionen auf Basis verschiedener Natriumchlorid-Konzentration bei der Elektrolyse (1,0 Gew.-%, 0,625 Gew.-%, 0,25 Gew.-%, 0,18 Gew.-%, 0,10 Gew.-% und 0,05 Gew.-%) auf einen Trockensubstanzgehalt von 20 % Kohlenhydrat verdünnt.

Diese Reaktionslösungen wurden dann jeweils mit einer Verweilzeit von 5,6 min in der Heizzone bei einer Temperatur von 165 °C (Temperatur des Thermoöls) umgesetzt.

| NaCl-Konzentration bei der Elektrolyse [%] | Chlorid-Konzentration in der Reaktionslösung bei der HMF-Synthese [mg/1] | Fructose-Umsatz [%] | HMF-Selektivität [%] | Bilanz [%] |
|---|---|---|---|---|
| 1,0 | 4683 | 29,6 | 86,3 | 97,3 |
| 0,625 | 3272 | 30,5 | 85,1 | 97,2 |
| 0,25 | 1203 | 30 | 86,7 | 97,3 |
| 0,18 | 993 | 29,7 | 84,1 | 96,0 |
| 0,10 | 408 | 30,4 | 79,9 | 95,2 |
| 0,05 | 326 | 29,0 | 80,6 | 96,1 |

Fructoseumsatz und HMF-Selektivität sind über einen Bereich weitestgehend unabhängig von der Chloridkonzentration.

### Beispiel 11: Effekt der Elektrolyse bei Verwendung von VE- und Leitungswasser

Im Rahmen dieser Versuche wurde jeweils eine Reaktionslösung mit einem Trockensubstanzgehalt von 20 % auf Basis eines Fructosesirups mit einer Fructosereinheit von 85 % und einem ursprünglichen Trockensubstanzgehalt von 75 % (F85/75) hergestellt. Zum Verdünnen wurde dabei einerseits reines VE-Wasser oder reines Leitungswasser und andererseits die jeweils bei der Elektrolyse entstandene katalytisch aktive Anolytfraktion von VE- oder Leitungswasser eingesetzt. Die daraus resultierenden Reaktionslösungen wurden dann in der Heizzone mit einer Verweilzeit von 5,6 min bei 169 °C umgesetzt. Durch regelmäßige Probenahme und Analyse der Proben mittels HPLC wurden Umsatz und Selektivitäten verfolgt und eine Kohlenstoffbilanz erstellt. Tabelle 1 zeigt die erhaltenen Ergebnisse.

**Tabelle 1:**

| **Versuch** | **2018MMR2** | **2018MMR4** | **2018MMR2** | **2017MMR166** |
|---|---|---|---|---|
| **Verdünnungswasser** | VE- Wasser | Anolytfraktion aus VE-Wasser-Elektrolyse | Leitungswasser | Anolytfraktion aus Leitungswasser-Elektrolyse |
| **pH-Wert der Reaktions-Lösung [-]** | 4,38 (reines VE-Wasser 6,24) | 4,2 | 7,62 | 2,4 |
| **Fructose-Umsatz** | 2,8 | 3,2 | 5,3 | 9,3 |
| **HMF-Selektivität [%]** | 76,3 | 84,6 | 29,2 | 82,8 |
| **Lävulinsäure-Selektivität [%]** | 0 | 0 | 0 | 0,98 |
| **Ameisensäure-Selektivität** | 0 | 0 | 4,45 | 2,47 |
| **Bilanz [%]** | 99,4 | 99,5 | 98,0 | 98,5 |

Die Ergebnisse zeigen eine deutliche Verbesserung sowohl hinsichtlich des Fructoseumsatzes als auch der HMF- und Nebenproduktselektivität bei Verwendung der jeweiligen Anolytfraktion von VE-Wasser als auch von Leitungswasser im Vergleich zu reinem VE- oder Leitungswasser.

### Beispiel 12: Effekt der Anolytfraktionkonzentration auf die HMF-Umsetzung

Im Rahmen dieser Versuchsreihe wurde zunächst eine katalytisch aktive Anolytfraktion auf Basis einer 0,18 %igen Natriumchloridlösung hergestellt. Ausgehend von dieser Anolytfraktion (100 % Anolytfraktion) wurden dann verschiedene Verdünnungen mit reinem VE-Wasser angesetzt (75 % Anolytfraktion / 25 % VE-Wasser, 50 % Anolytfraktion / 50 % VE-Wasser und 25 % Anolytfraktion / 75 % VE-Wasser). Diese Mischungen wurden dann jeweils zur Herstellung einer Reaktionslösung mit einem Trockensubstanzgehalt von 20 % TS auf Basis eines Fructosesirups mit 85 % Fructosereinheit und einem ursprünglichen Trockensubstanzgehalt von 75 % eingesetzt, die dann unter gleichen Reaktionsbedingungen (Verweilzeit 5,6 min in der Heizzone, Temperatur 169 °C) umgesetzt wurden. Durch regelmäßige Probenahme und Analyse der Proben mittels HPLC wurden Umsatz und Selektivitäten verfolgt und eine Kohlenstoffbilanz erstellt. Tabelle 2 zeigt die erhaltenen Ergebnisse.

**Tabelle 2:**

| **Versuch** | **1** | **2** | **3** | **4** |
|---|---|---|---|---|
| **Verdünnungswasser** | 100 % Anolytfraktion | 75 % Anolytfraktion / 25 % VE-Wasser | 50 % Anolytfraktion / 50 % VE-Wasser | 25 % Anolytfraktion / 75 % VE-Wasser |
| **pH der Reaktionslösung [-]** | 2,2 | 2,4 | 2,6 | 2,9 |
| **Fructose-Umsatz** | 20,1 | 14,4 | 10,4 | 5,2 |
| **HMF -Selektivität [%]** | 87,9 | 89,0 | 91,9 | 100 |
| **Lävulinsäure-Selektivität [%]** | 1,7 | 1,3 | 0,9 | 0 |
| **Ameisensäure-Selektivität [%]** | 3,3 | 1,6 | 2,2 | 0 |
| **Bilanz [%]** | 97,7 | 98,1 | 99,6 | 100 |

Die Ergebnisse zeigen eine deutliche Abhängigkeit sowohl des Fructoseumsatzes als auch der HMF- und Nebenprodukselektivitäten sowie Kohlenstoffbilanz von der Konzentration der Anolytfraktion. Im Vergleich zu reinem VE-Wasser bzw. der Anolytfraktion von VE-Wasser (siehe Tabelle 1) zeigen alle Versuche eine deutliche Verbesserung insbesondere hinsichtlich der Selektivität.

### Beispiel 13: Sauerstoffgehalt verschiedener Anolytfraktionen

Unterschiedliche Anolytfraktionen wurden hergestellt und hinsichtlich ihres Sauerstoffgehalts und pH-Werts mittels eines Sauerstoffmessgeräts 4100e Mettler Toledo untersucht. Zum Vergleich wurde der Sauerstoffgehalt von vollentsalztem Wasser bestimmt.

| **Lösung** | **pH** | **Temperatur** | **O₂-Gehalt (mg/I)** |
|---|---|---|---|
| **VE-Wasser** | 6,24 | 21,3 | 9,3 |
| **Anolytfraktion aus VE-Wasser** | 3,0 | 23,1 | 19,4 |
| **Anolytfraktion aus 0,3 Gew.-% NaNO₃** | 2,31 | 21,2 | 20,6 |
| **Anolytfraktion aus 0,5 Gew.-% NaNO₃** | 1,98 | 21,5 | 27,5 |
| **Anolytfraktion aus 1,0 Gew.-% NaNO₃** | 1,92 | 21,2 | 25,8 |
| **Anolytfraktion aus 2,0 Gew.-% NaNO₃** | 1,87 | 22,2 | 25,6 |
| **Anolytfraktion aus 0,2 Gew.-% NaCl** | 2,14 | 21,5 | 19,4 |
| **Anolytfraktion aus 0,5 Gew.-% NaCl** | 1,98 | 21,6 | 26,81 |
| **Anolytfraktion aus 1,0 Gew.-% NaCl** | 2,02 | 21,4 | 24,2 |

Die in der Tabelle dargestellten Ergebnisse zeigen, dass alle Anolytfraktionen einen deutlich erhöhten Sauerstoffgehalt gegenüber nicht elektrolysiertem vollentsalztem Wasser aufweisen.

## Patentansprüche

1. Verfahren zur Herstellung von 5-Hydroxymethylfurfural (HMF) umfassend die Schritte:
a) Bereitstellen einer Fructose-haltigen Komponente und einer katalytisch aktiven Anolytfraktion, welche durch Elektrolyse von Wasser hergestellt worden ist, wobei die Elektrolyse von Wasser in einer Elektrolysezelle durchgeführt wurde, worin die Kathode von der Anode getrennt ist, wobei die Anolytfraktion aus dem Anodenraum der Elektrolysezelle nach Durchführung der Elektrolyse entnommen wurde und wobei die katalytisch aktive Anolytfraktion einen Sauerstoffgehalt oberhalb der Sättigungskonzentration aufweist,
b) Vermischen der Fructose-haltigen Komponente und der katalytisch aktiven Anolytfraktion zum Erhalt einer Reaktionslösung,
c) Umsetzen der in der Reaktionslösung vorhandenen Fructose zu HMF bei einer Temperatur von 90 °C bis 200 °C zum Erhalten einer flüssigen HMF-haltigen Produktmischung und
d) Erhalten einer flüssigen HMF-haltigen Produktmischung.

2. Verfahren nach Anspruch 1, wobei das Wasser für die Elektrolyse vollentsalztes Wasser ist.

3. Verfahren nach Anspruch 1, wobei das Wasser für die Elektrolyse ein Salz aufweist, ausgewählt aus der Gruppe bestehend aus Alkalihalogeniden, Erdalkalihalogeniden, Alkalinitraten, Erdalkalinitraten, Alkalisulfaten, Erdalkalisulfaten, Citraten, Acetaten, Tartraten, Oxalaten, Glycolaten, Gluconaten und Gemische davon.

4. Verfahren nach Anspruch 3, wobei das Wasser für die Elektrolyse 0,01 bis 2,5 Gew.-% Salz (bezogen auf das Gesamtgewicht des Wassers) aufweist.

5. Verfahren nach einem der vorangehenden Ansprüche, wobei der pH-Wert der katalytisch aktiven Anolytfraktion 1,5 bis 4,5, bevorzugt 2 bis 3, beträgt.

6. Verfahren nach einem der vorangehenden Ansprüche, wobei die Fructose-haltige Komponente eine feste Fructose-haltige Komponente, insbesondere Fructose, oder eine flüssige Fructose-haltige Komponente, insbesondere ein Fructosesirup oder eine Fructoselösung ist.

7. Verfahren nach einem der vorangehenden Ansprüche, wobei in Verfahrensschritt b) eine Reaktionslösung mit einem Kohlenhydratgehalt von 5 bis 50 Gew.-% (Trockensubstanz Kohlenhydrat in Bezug auf Gesamtgewicht Reaktionslösung) erhalten und in Verfahrensschritt c) eingesetzt wird.

8. Verfahren nach einem der vorangehenden Ansprüche, wobei in Verfahrensschritt b) eine Reaktionslösung mit einem Fructosegehalt von 40 bis 100 Gew.-% (Trockensubstanz Fructose in Bezug auf Trockensubstanz des Kohlenhydratanteils der Reaktionslösung) erhalten und in Verfahrensschritt c) eingesetzt wird.

9. Verfahren nach einem der vorangehenden Ansprüche, wobei das Verhältnis von Kohlenhydratgehalt (Trockensubstanz) der Fructose-haltigen Komponente zu katalytisch aktiver Anolytfraktion (Gesamtgewicht) in der Reaktionslösung 0,01 - 2,5 beträgt.

10. Verfahren nach einem der vorangehenden Ansprüche, wobei das Verhältnis von Fructosegehalt (TS) der Fructose-haltigen Komponente zu katalytisch aktiver Anolytfraktion (Gesamtgewicht) in der Reaktionslösung 0,01 - 2,5 beträgt.

11. Verfahren nach einem der vorangehenden Ansprüche, wobei die in Schritt a) bereitgestellte Fructose-haltige Komponente, die Anolytfraktion oder beide vor Schritt b) auf eine Temperatur von 90 °C bis 200 °C eingestellt werden oder wobei die in Schritt b) erhaltene Reaktionslösung auf eine Temperatur von 90 °C bis 200 °C eingestellt wird.

12. Verfahren nach einem der vorangehenden Ansprüche, wobei das Verfahren so geführt wird, dass in Verfahrensschritt c) ein Fructoseumsatz von 1 bis 50 mol-% erreicht wird.

13. Verfahren nach einem der vorangehenden Ansprüche, wobei das Verfahren so eingestellt wird, dass in Verfahrensschritt c) eine HMF-Selektivität von 60 bis 100 mol-% erhalten wird.

14. Verfahren nach einem der vorangehenden Ansprüche, wobei das Verfahren kontinuierlich durchgeführt wird.

15. Verfahren nach einem der vorangehenden Ansprüche, wobei in dem Verfahren abgesehen von der katalytisch aktiven Anolytfraktion keine weitere katalytisch aktive Komponente verwendet wird.

16. Verfahren nach einem der vorangehenden Ansprüche, umfassend den Schritt:
e) Abkühlen der flüssigen HMF-Produktmischung auf eine Temperatur von 20° bis 80° C.

17. Verfahren nach einem der vorangehenden Ansprüche, umfassend den Schritt:
f) Filtration, Entfärbung und/oder Reinigung der flüssigen HMF-Produktmischung.

18. Verfahren nach einem der vorangehenden Ansprüche, umfassend den Schritt:
g) Einstellen der flüssigen HMF-Produktmischung auf einen Trockensubstanzgehalt von 20 bis 70 Gew.-%.

19. Verfahren nach einem der vorangehenden Ansprüche, umfassend die Schritte:
h) Aufreinigen der flüssigen HMF-Produktmischung mittels Chromatographie, Ultra- und/oder Nanofiltration, Extraktion mit einem geeigneten Extraktionsmittel, Adsorption an ein geeignetes Material und anschließender gezielter Desorption und/oder Elektrodialyse zur Abtrennung mindestens einer HMF-Fraktion, und
i) Erhalten mindestens einer HMF-Fraktion.

20. Verfahren nach Anspruch 19, wobei die flüssige HMF-Produktmischung in Schritt h) mittels Chromatographie in mindestens vier Fraktionen, umfassend eine HMF-Fraktion, eine Glucose-Fraktion, eine Fructose-Fraktion und eine organische Säuren Fraktion aufgetrennt und in Schritt i) mindestens eine HMF-Fraktion, eine Glucose-Fraktion, eine Fructose-Fraktion und eine organische Säuren Fraktion erhalten wird.

21. Verfahren nach Anspruch 20, wobei die in Verfahrensschritt i) erhaltene Fructose-Fraktion in den Schritt a) rückgeführt wird.

22. Verfahren nach einem der vorangehenden Ansprüche 20 bis 21, wobei die in Verfahrensschritt i) erhaltene Glucose-Fraktion für die Ethanolherstellung eingesetzt wird.

23. Verfahren nach einem der vorangehenden Ansprüche 20 bis 22, wobei die in Verfahrensschritt i) erhaltene organische Säuren-Fraktion zur Isolierung von Lävulin- und Ameisensäure eingesetzt wird.

24. Verfahren nach einem der vorangehenden Ansprüche 19 bis 23, wobei die in Verfahrensschritt i) erhaltene HMF-Fraktion direkt und ohne die Notwendigkeit weiterer Aufreinigung in einem weiteren Schritt zu 2,5-Furandicarbonsäure (FDCA) oxidiert wird.

## Claims

1. Method for the production of 5-hydroxymethylfurfural (HMF) comprising the following steps:
a) providing a fructose-containing component and a catalytically active anolyte fraction which has been produced by electrolysis of water, wherein the electrolysis of water was conducted in an electrolysis cell, wherein the cathode is separated from the anode, wherein after the electrolysis has been carried out the anolyte fraction was removed from the anode compartment of the electrolysis cell and wherein the anolyte fraction has an oxygen content above the saturation concentration,
b) mixing the fructose-containing component and the catalytically active anolyte fraction to obtain a reaction solution,
c) converting the fructose present in the reaction solution to HMF at a temperature of 90 °C to 200 °C to obtain a liquid HMF-containing product mixture and
d) obtaining a liquid HMF-containing product mixture.

2. Method according to claim 1, wherein the water for the electrolysis is fully demineralized water.

3. Method according to claim 1, wherein the water for the electrolysis comprises a salt selected from the group consisting of alkaline halides, alkaline earth halides, alkaline nitrates, alkaline earth nitrates, alkaline sulfates, alkaline earth sulfates, citrates, acetates, tartrates, oxalates, glycolates, gluconates and mixtures thereof.

4. Method according to claim 3, wherein the water for the electrolysis comprises 0.01 to 2.5 wt.-% of salt (based on the total weight of the water).

5. Method according to any of the preceding claims, wherein the pH of the catalytically active anolyte fraction is 1.5 to 4.5, preferably 2 to 3.

6. Method according to any of the preceding claims, wherein the fructose-containing component is a solid fructose-containing component, in particular fructose, or a liquid fructose-containing component, in particular a fructose syrup or a fructose solution.

7. Method according to any of the preceding claims, wherein in method step b) a reaction solution with a carbohydrate content of 5 to 50 wt.-% (dry matter carbohydrate in relation to the total weight of reaction solution) is obtained and used in method step c).

8. Method according to any of the preceding claims, wherein in method step b) a reaction solution with a fructose content of 40 to 100 wt.-% (dry matter fructose in relation to dry matter of the carbohydrate content of the reaction solution) is obtained and used in method step c).

9. Method according to any of the preceding claims, wherein the ratio of the carbohydrate content (dry matter) of the fructose-containing component to the catalytically active anolyte fraction (total weight) in the reaction solution is 0.01-2.5.

10. Method according to any of the preceding claims, wherein the ratio of fructose content (DM) of the fructose-containing component to catalytically active anolyte fraction (total weight) in the reaction solution is 0.01-2.5.

11. Method according to any of the preceding claims, wherein the fructose-containing component provided in step a), the anolyte fraction or both are set to a temperature of 90 °C to 200 °C before step b) or wherein the reaction solution obtained in step b) is set to a temperature of 90 °C to 200 °C.

12. Method according to any of the preceding claims, wherein the process is carried out such that a fructose conversion of 1 to 50 mol-% is achieved in method step c).

13. Method according to any of the preceding claims, wherein the method is set so that in method step c) an HMF selectivity of 60 to 100 mol-% is obtained.

14. Method according to any of the preceding claims, wherein the method is carried out continuously.

15. Method according to any of the preceding claims, wherein apart from the catalytically active anolyte fraction, no further catalytically active component is used in the process.

16. Method according to any of the preceding claims, comprising the following step:
e) cooling the liquid HMF product mixture to a temperature of 20 to 80 °C.

17. Method according to any of the preceding claims, comprising the following step:
f) filtration, decolorization and/or purification of the liquid HMF product mixture.

18. Method according to any of the preceding claims, comprising the following step:
g) setting the liquid HMF product mixture to a dry matter content of 20 to 70 wt.%.

19. Method according to any of the preceding claims, comprising the following steps:
h) purification of the liquid HMF product mixture using chromatography, ultraand/or nanofiltration, extraction with a suitable extractant, adsorption on a suitable material and subsequent targeted desorption and/or electrodialysis to separate at least one HMF fraction, and
i) obtaining at least one HMF fraction.

20. Method according to claim 19, wherein the liquid HMF product mixture is separated in step h) using chromatography into at least four fractions comprising an HMF fraction, a glucose fraction, a fructose fraction and an organic acid fraction and in step i) at least an HMF fraction, a glucose fraction, a fructose fraction and an organic acid fraction is obtained.

21. Method according to claim 20, wherein the fructose fraction obtained in method step i) is recycled into step a).

22. Method according to any of the preceding claims 20 to 21, wherein the glucose fraction obtained in method step i) is used for the production of ethanol.

23. Method according to any of the preceding claims 20 to 22, wherein the organic acid fraction obtained in method step i) is used to isolate levulinic and formic acid.

24. Method according to any of the preceding claims 19 to 23, wherein the HMF fraction obtained in method step i) is oxidized directly and is oxidized in a further step to 2,5-furandicarboxylic acid (FDCA) without the need for further purification.

## Revendications

1. Procédé pour la production de 5-hydroxyméthylfurfural (HMF) comprenant les étapes suivantes :
a) fournir un composant contenant du fructose et une fraction anolyte catalytiquement active qui a été produite par électrolyse de l'eau, dans lequel l'électrolyse de l'eau a été réalisée dans une cellule d'électrolyse, dans laquelle la cathode est séparée de l'anode, dans laquelle après que l'électrolyse ait été réalisée, la fraction anolyte a été retirée du compartiment anodique de la cellule d'électrolyse et dans laquelle la fraction anolyte a une teneur en oxygène supérieure à la concentration de saturation,
b) mélanger le composant contenant du fructose et la fraction anolyte catalytiquement active pour obtenir une solution de réaction,
c) convertir le fructose présent dans la solution réactionnelle en HMF à une température de 90 °C à 200 °C pour obtenir un mélange de produits liquide contenant du HMF et
d) l'obtention d'un mélange de produits liquide contenant du HMF.

2. Le procédé selon la revendication 1, dans lequel l'eau pour l'électrolyse est de l'eau entièrement déminéralisée.

3. Le procédé selon la revendication 1, dans lequel l'eau pour l'électrolyse comprend un sel choisi dans le groupe constitué par les halogénures alcalins, les halogénures alcalino-terreux, les nitrates alcalins, les nitrates alcalino-terreux, les sulfates alcalins, les sulfates alcalino-terreux, les citrates, les acétates, les tartrates, les oxalates, les glycolates, les gluconates et leurs mélanges.

4. Procédé selon la revendication 3, dans lequel l'eau pour l'électrolyse comprend 0,01 à 2,5 % en poids de sel (sur la base du poids total de l'eau).

5. Le procédé selon l'une quelconque des revendications précédentes, dans lequel le pH de la fraction anolyte catalytiquement active est de 1,5 à 4,5, préférablement de 2 à 3.

6. Le procédé selon l'une quelconque des revendications précédentes, dans lequel le composant contenant du fructose est un composant solide contenant du fructose, en particulier du fructose, ou un composant liquide contenant du fructose, en particulier un sirop de fructose ou une solution de fructose.

7. Le procédé selon l'une quelconque des revendications précédentes, dans lequel, à l'étape b) du procédé, une solution réactionnelle ayant une teneur en glucides de 5 à 50 % en poids (glucides de la matière sèche par rapport au poids total de la solution réactionnelle) est obtenue et utilisée à l'étape c) du procédé.

8. Le procédé selon l'une quelconque des revendications précédentes, dans lequel, dans l'étape b) du procédé, une solution réactionnelle ayant une teneur en fructose de 40 à 100 % en poids (fructose en matière sèche par rapport à la matière sèche de la teneur en glucides de la solution réactionnelle) est obtenue et utilisée dans l'étape c) du procédé.

9. Le procédé selon l'une quelconque des revendications précédentes, dans lequel le rapport entre la teneur en glucides (matière sèche) du composant contenant du fructose et la fraction anolyte catalytiquement active (poids total) dans la solution réactionnelle est de 0,01 à 2,5.

10. Le procédé selon l'une quelconque des revendications précédentes, dans lequel le rapport de la teneur en fructose (MS) du composant contenant du fructose sur la fraction anolyte catalytiquement active (poids total) dans la solution réactionnelle est de 0,01 à 2,5.

11. Le procédé selon l'une quelconque des revendications précédentes, dans lequel le composant contenant du fructose fourni à l'étape a), la fraction anolyte ou les deux sont réglés à une température de 90 °C à 200 °C avant l'étape b) ou dans lequel la solution réactionnelle obtenue à l'étape b) est réglée à une température de 90 °C à 200 °C.

12. Le procédé selon l'une quelconque des revendications précédentes, dans lequel le procédé est mis en œuvre de telle sorte qu'une conversion du fructose de 1 à 50 % en moles est achevée dans l'étape c) du procédé.

13. Le procédé selon l'une quelconque des revendications précédentes, dans lequel le procédé est mis en œuvre de telle sorte que dans l'étape c) du procédé, une sélectivité en HMF de 60 à 100 % en moles est obtenue.

14. Le procédé selon l'une quelconque des revendications précédentes, dans lequel le procédé est mis en œuvre de manière continue.

15. Le procédé selon l'une quelconque des revendications précédentes, dans lequel, à part la fraction anolyte catalytiquement active, aucun autre composant catalytiquement actif n'est utilisé dans le procédé.

16. Le procédé selon l'une quelconque des revendications précédentes, comprenant l'étape suivante :
e) refroidissement du mélange liquide de produits HMF à une température comprise entre 20 et 80 °C.

17. Le procédé selon l'une quelconque des revendications précédentes, comprenant l'étape suivante :
f) filtration, décoloration et/ou purification du mélange liquide de produits HMF.

18. Le procédé selon l'une quelconque des revendications précédentes, comprenant l'étape suivante :
g) regler du mélange liquide de produits HMF à une teneur en matière sèche de 20 à 70 % en poids.

19. Le procédé selon l'une quelconque des revendications précédentes, comprenant les étapes suivantes :
h) purification du mélange liquide de produits HMF en utilisant la chromatographie, l'ultrafiltration et/ou la nanofiltration, l'extraction avec un agent d'extraction approprié, l'adsorption sur un matériau approprié et ensuite la désorption ciblée et/ou l'électrodialyse pour séparer au moins une fraction HMF, et
i) obtenir au moins une fraction HMF.

20. Le procédé selon la revendication 19, dans lequel le mélange liquide de produits HMF est séparé à l'étape h) en utilisant la chromatographie en au moins quatre fractions comprenant une fraction HMF, une fraction glucose, une fraction fructose et une fraction acide organique et à l'étape i) au moins une fraction HMF, une fraction glucose, une fraction fructose et une fraction acide organique est obtenue.

21. Le procédé selon la revendication 20, dans lequel la fraction de fructose obtenue dans l'étape i) du procédé est recyclée dans l'étape a).

22. Le procédé selon l'une quelconque des revendications précédentes 20 à 21, dans lequel la fraction de glucose obtenue à l'étape i) du procédé est utilisée pour la production d'éthanol.

23. Le procédé selon l'une quelconque des revendications précédentes 20 à 22, dans lequel la fraction d'acide organique obtenue à l'étape i) du procédé est utilisée pour isoler les acides lévulinique et formique.

24. Le procédé selon l'une quelconque des revendications précédentes 19 à 23, dans lequel la fraction HMF obtenue dans l'étape i) du procédé est oxydée directement et est oxydée dans une étape supplémentaire en acide 2,5-furandicarboxylique (FDCA) sans nécessité de purification supplémentaire.
